(19) **Europäisches Patentamt / European Patent Office / Office européen des brevets**

(11) **EP 3 179 921 B1**

(12) ## EUROPEAN PATENT SPECIFICATION

(45) Date of publication and mention
of the grant of the patent:
**05.10.2022 Bulletin 2022/40**

(21) Application number: **15832136.4**

(22) Date of filing: **10.08.2015**

(51) International Patent Classification (IPC):
*A61B 10/02* (2006.01)   *A61B 5/00* (2006.01)
*A61B 5/06* (2006.01)   *A61B 10/00* (2006.01)

(52) Cooperative Patent Classification (CPC):
**A61B 10/0041; A61B 5/0053; A61B 5/062;
A61B 5/067; A61B 5/4312; A61B 5/6848;
A61B 10/0233; A61B 10/0283;** A61B 5/7242

(86) International application number:
**PCT/US2015/044469**

(87) International publication number:
**WO 2016/025389 (18.02.2016 Gazette 2016/07)**

(54) **FINE NEEDLE ELASTOGRAPHY DEVICE AND SYSTEM FOR THE MEASUREMENT OF MATERIAL PROPERTIES**

FEINNADELELASTOGRAFIEVORRICHTUNG UND SYSTEM ZUR MESSUNG VON MATERIALEIGENSCHAFTEN

DISPOSITIF ET SYSTÈME D'ÉLASTOGRAPHIE PAR AIGUILLE FINE POUR LA MESURE DES PROPRIÉTÉS D'UN MATÉRIAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.08.2014 US 201462035976 P**

(43) Date of publication of application:
**21.06.2017 Bulletin 2017/25**

(73) Proprietors:
• **The Regents of the University of California**
  **Oakland, CA 94607 (US)**
• **Gimzewski, James K.**
  **Topanga, California 90290 (US)**
• **Sharma, Shivani**
  **El Segundo, California 90245 (US)**
• **Wilkinson, Paul R.**
  **El Segundo, California 90245 (US)**
• **Ragavendra, Nagesh**
  **Malibu, California 90265 (US)**
• **Rao, Jianyu**
  **Los Angeles, California 90095 (US)**
• **Wickramaratne, M. Dayan J.**
  **Los Angeles, California 90024 (US)**

(72) Inventors:
• **GIMZEWSKI, James K.**
  **Topanga, California 90290 (US)**
• **SHARMA, Shivani**
  **Los Angeles, California 90025 (US)**
• **WILKINSON, Paul R.**
  **El Segundo, California 90245 (US)**
• **RAGAVENDRA, Nagesh**
  **Malibu, California 90265 (US)**
• **RAO, JianYu**
  **Los Angeles, California 90095 (US)**
• **WICKRAMARATNE, M. Dayan J.**
  **Los Angeles, California 90024 (US)**

(74) Representative: **Forresters IP LLP
Skygarden
Erika-Mann-Straße 11
80636 München (DE)**

(56) References cited:
WO-A1-2009/025779   WO-A1-2009/029627
WO-A1-2014/097301   WO-A2-2004/082468
US-A1- 2004 249 268   US-A1- 2007 191 733
US-A1- 2011 004 104   US-A1- 2011 054 354
US-A1- 2013 291 658   US-A1- 2014 039 314
US-A1- 2014 142 429   US-B2- 7 731 661

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**CROSS-REFERENCE TO RELATED PATENT APPLICATIONS**

This application claims the benefit of U.S. Provisional Patent Application 62/035,976 filed August 11, 2014 to Gimzewski et al., titled "FINE NEEDLE ELASTOGRAPHY DEVICE AND SYSTEM FOR THE MEASUREMENT OF MATERIAL PROPERTIES".

**BACKGROUND**

New ways to identify early stage cancer are desirable.

For example, thyroid cancer is one of the most curable forms of cancer. It is the most common endocrine cancer, accounting for 1.0%-1.5% of all new cancers diagnosed each year in United States. Largely due to lack of awareness and delay in diagnosis, thyroid cancer affected about 60,220 people and resulted in about 1,850 deaths in the United States in 2013 alone, with 60% of cases diagnosed at an intermediate or high-risk stage, when it is difficult to treat the cancer.

For another example, one of the most common forms of cancer, and a leading cause of cancer deaths in women in the United States, is breast cancer. In premenopausal women, one in twelve identified lumps is malignant. In postmenopausal women, it is approximately one in two. Thus, efficient evaluation and prompt diagnosis are needed to rule out malignancy and minimize unnecessary testing and invasive surgical procedures, and there is also a need to minimize the prevalent occurrence of over-diagnosis and overtreatment of breast cancer. Breast cancer is typically diagnosed in a three-stage process of clinical breast examination, imaging, and tissue sampling. However, palpable breast masses are common and usually benign (e.g., fibroadenomas and cysts), so at the clinical breast examination stage there is over-diagnosis of areas of concern.

In addition to palpation, FNA (Fine Needle Aspiration) cytology (FNAC) may be used, and is currently the most accurate and sensitive diagnostic tool for an initial screening of patients in United States. However, FNAC suffers from pitfalls related to specimen adequacy, sampling techniques, skill of the physician performing the aspiration, experience of the pathologist interpreting the aspirate, and overlapping cytologic features between benign and malignant follicular neoplasms. For example, FNAC sensitivity and specificity values of thyroid FNA vary from 65% to 98% and from 73% to 100%, respectively. A large reason for such a wide range of sensitivity and specificity is how cytopathologists handle the category of "suspicious," and how they define false-positive and false-negative results. Additionally, FNAC analysis involves sample preparation and imaging capabilities, and is constrained in quantitative identification of malignant tumors.

More recently, elastography methods have been used to determine a relationship between different structures and their respective tissue elasticity that can aid in diagnosing malignant tumors. Exemplary elastography devices are disclosed in WO2009/029627 A1 and WO2014/097301 A1. Elastography provides a way to qualitatively image tissue stiffness, and may provide additional improved sensitivity, objectivity, or details such as stiffness patterns (e.g., as compared to manual palpation or FNAC). However, elastography is expensive, qualitative and involves specialized instrumentation and operational skills.

Thus there remains an unmet need for quantitative, cost effective and easy-to-use early cancer diagnosis methods. It is against this background that a need arose to develop the embodiments described herein.

**SUMMARY**

The present invention relates to an elastography system and is set out in the set of appended claims.

**BRIEF DESCRIPTION OF THE DRAWINGS**

FIG. 1 is an illustration of an example of a fine needle elastography (FNE) system according to an embodiment of the present disclosure.
FIG. 2 is an illustration of an example of a computing device according to an embodiment of the present disclosure.
FIG. 3A is an illustration of an example of a fine needle elastography (FNE) device according to an embodiment of the present disclosure.
FIG. 3B is a representation the FNE device of FIG. 3A according to an embodiment of the present disclosure.
FIG. 3C is an illustration of holding the FNE device of FIG. 3A.
FIG. 4 is a representation the FNE device of FIG. 3A according to an embodiment of the present disclosure.
FIG. 5 is a plot of charge versus force for an FNE device according to an embodiment of the present disclosure.

FIG. 6 is a plot of force versus distance during insertion of an FNE device according to an embodiment of the present disclosure.

FIG. 7A is a plot of change in force versus distance during insertion of an FNE device according to an embodiment of the present disclosure.

FIG. 7B is a bar chart representing force heterogeneity of the curves in the plot of FIG. 7A.

FIG. 7C is a plot of a derivative of force versus distance during insertion of an FNE device according to an embodiment of the present disclosure.

FIG. 7D is a bar chart representing stiffness heterogeneity of the curves in the plot of FIG. 7C.

FIG. 8A is an illustration of inserting into a neck phantom an FNE device according to an embodiment of the present disclosure.

FIG. 8B is an ultrasound image during insertion into a neck phantom an FNE device according to an embodiment of the present disclosure.

FIG. 9A is a plot of force versus distance during insertion of an FNE device according to an embodiment of the present disclosure.

FIG. 9B is an ultrasound image of a portion of a neck phantom.

FIG. 10A is a plot of force versus distance during insertion of an FNE device according to an embodiment of the present disclosure.

FIG. 10B is an ultrasound image of a portion of a neck phantom.

FIG. 11 is a plot of change in force versus change in distance during insertion of an FNE device according to an embodiment of the present disclosure.

FIG. 12A is a plot of change in force versus distance during insertion of an FNE device according to an embodiment of the present disclosure.

FIG. 12B is a bar chart representing force heterogeneity of the curves in the plot of FIG. 12A.

FIG. 12C is a plot of a derivative of force versus change in distance during insertion of an FNE device according to an embodiment of the present disclosure.

FIG. 12D is a bar chart representing stiffness heterogeneity of the curves in the plot of FIG. 12C.

FIG. 13A is an illustration of an example of an FNE device according to an embodiment of the present disclosure.

FIG. 13B is a block diagram representation of the FNE device of FIG. 13A.

FIG. 14A is a schematic representation of a portion of the FNE device of FIG. 13A.

FIG. 14B is an image of a prototype of a charge amplifier of the FNE device of FIG. 13A.

FIG. 15 is plot of force versus needle depth during insertion of an FNE device according to an embodiment of the present disclosure.

FIG. 16 is an illustration of a technique for performing an *in vivo* mapping of an irregularity in breast tissue.

## DETAILED DESCRIPTION

Acronyms, abbreviations, and symbols used in the present disclosure include those shown in the three columns of Table 1.

**Table 1**

| | | |
|---|---|---|
| FNA (fine needle aspiration) | Hertz (Hz) | pico (p) (e.g., picofarad (pF)) |
| FNAB (FNA biopsy) | Newton (N) | nano (n) (e.g., nanovolt (nV)) |
| FNAC (FNA cytology) | Coulomb (C) | micro ($\mu$) (e.g., micrometer ($\mu$m)) |
| FNE (fine needle elastography) | Farad (F) | milli (m) (e.g., millimeter (mm); millinewton (mN); milliliter (mL)) |
| UE (ultrasound elastography) | Ohm ($\Omega$) | kilo (k) (e.g., kilohertz (kHz); kilonewton (kN); kilopascal (kPa)) |
| AFM (atomic force microscopy) | Volts (V) | mega (M) (e.g., megahertz (MHz); megaohm (M$\Omega$)) |
| second (s) | Amperes (A) | giga (G) (e.g., gigahertz (GHz)) |
| meter (m) | Pascals (P) | three-dimensional (3D) |
| gram (g) | | |
| liter (1) | | |

Different materials have different material properties, and investigation of the material properties of a material provides information about the material type. With respect to biological material, investigation of the material properties of the biological material provides information about the biological material type. For example, irregularities such as cancers, cysts, and lipomas may be detected by examining biological material properties. Additionally, investigation of a biological material may be used to confirm that irregularities are not present.

In the present disclosure, the term "tissue" is used to represent "biological material" for simplicity; however, it is to be understood that "biological material" encompasses other materials beyond tissue. Further, the concepts of the present application are extendable to non-biological materials.

One category of material property is resistive force. As described in the present disclosure, a needle may be used to detect resistive force by inserting the needle into a tissue and quantitatively measuring resistive forces experienced by the needle. Examples of resistive forces in tissue include inertial force, elastic restoring force, friction force, and cutting force. Measured resistive force allows an irregularity to be mapped, and capture tissue material properties that change within the irregularity and at its boundary. Accurate mapping of an irregularity, for example, allows for better precision in diagnosis and treatment or removal (e.g., excision) of the irregularity.

As described in the present disclosure, an FNE device, an FNE system and FNE techniques are used to measure resistive force of tissue. The FNE device includes a force sensor and a needle. In one or more embodiments, the force sensor is a piezoelectric crystal mounted between the needle and its mounting base. Such a device can be used to map subsurface variations in material properties with a resolution and penetration depth that exceeds existing devices, including medical ultrasound. Improved resolution improves speed and accuracy of diagnosis. The disclosed FNE device, system and techniques can therefore replace medical ultrasound in a number of clinical applications. Further, the disclosed FNE device, system and techniques are particularly useful where other equipment is cost prohibitive or not available, or where technicians or pathologists are not available.

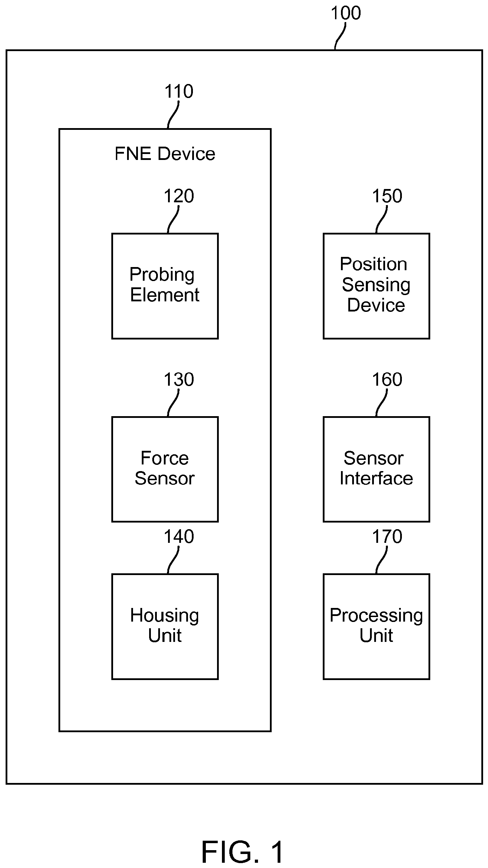

FIG. 1 illustrates an example of an FNE system 100 according to one or more embodiments of the present disclosure. System 100 includes an FNE device 110, which in turn includes a probing element 120, a force sensor 130 and a housing unit 140. The FNE system 100 optionally further includes a position sensing device 150, a sensor interface 160, and a processing unit 170, one or more of which may be included within housing unit 140, or may be external to housing unit 140.

Probing element 120 is a high aspect ratio probing element (e.g., an aspect ratio of about 2 or greater, about 3 or greater, about 4 of greater, or about 5 or greater, and up about 10 or greater, or up to about 20 or greater). Examples of probing element 120 include a needle, a tube, or a wire, such as an FNAC biopsy hypodermic needle, a custom needle from capillary tubing, a custom solid needle, a nanowire, and a nanotube, among others.

Force sensor 130 is connected to probing element 120. Examples of force sensor 130 include a piezoelectric crystal, a strain gauge, and a rigid flexural element with a displacement measuring sensor (e.g., a capacitive displacement sensor, an optical displacement sensor, or an inductive displacement sensor).

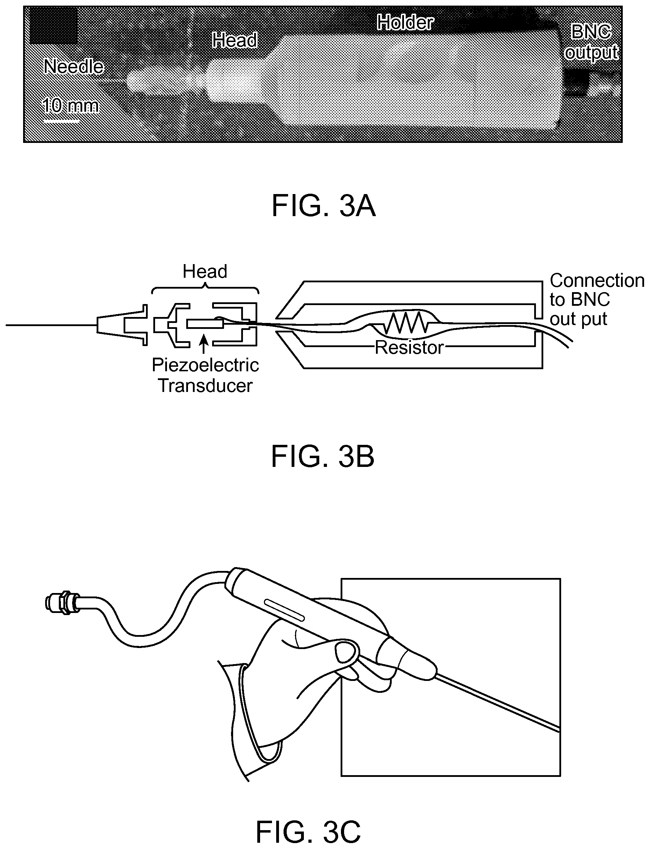

Housing unit 140 includes attachment areas to which, or enclosure areas within which, probing element 120 and force sensor 130 can be disposed or mounted. According to the invention ,probing element 120 and force sensor 130 are mounted so as to have a fixed spatial relationship or distance relative to one another and a fixed spatial relationship or distance relative to housing unit 140, which facilitates speed and accuracy of information processing. In one or more embodiments, housing unit 140 is implemented as a single component. In one or more embodiments, housing unit 140 is implemented as multiple components, such as a head portion and a holder portion. In such embodiments, for example, force sensor 130 and probing element 120 may be mounted to the head portion, and one or both of force sensor 130 and probing element 120 may be removably mounted. The head portion may then be placed in the holder. Such a housing unit 140 with a head portion and a holder portion provides for handheld operation (see, for example, Figure 3C).

In one or more embodiments, FNE device 110 is implemented in a modular architecture, which allows for removal of probing element 120 and may allow for removal of force sensor 130. Such embodiments provide, for example, sterilization or replacement of probing element 120 or force sensor 130. Thus, for example, mass-produced needles may be used as probing element 120, thereby reducing cost and improving hygiene. Additionally, the ability to replace force sensor 130 provides for upgrading force sensor 130, or changing one type of force sensor 130 to another type of force sensor 130 for a particular investigation.

Position sensing device 150 is optional, and senses or establishes an absolute position, a relative position, or a displacement of position. Position can refer to coordinates within a three-dimensional (3D) space as well as orientation with respect to a line or plane. Displacement may be determined, for example, according to change in absolute or relative position, or by position in combination with velocity and/or acceleration. Examples of position sensing device 150 include one of, or a combination of, a gyroscope, an accelerometer, an inertial measurement unit, an ultrasound displacement sensor, a magnetic displacement sensor, an electric displacement sensor, or other sensor. Position sensing device 150 can be implemented, for example, as an encoded stage to establish a position or a path of the probing element.

Sensor interface 160 is optional, and gathers information from position sensing device 150, to provide sensor information to another component such as processing unit 170. Sensor interface 160 may include analog or digital components, or a combination thereof. For embodiments in which position sensing device 150 outputs digital data, sensor interface 160 includes a digital interface. For embodiments in which position sensing device 150 outputs analog data, sensor interface 160 includes an analog interface. An analog interface may include an analog-to-digital converter (ADC); however, this is not necessarily the case. Sensor interface 160 provides information to other components in a form receivable by the other components. For example, if the information is provided to an analog input of processing unit 170, the form output by sensor interface 160 may be analog; whereas if the information is provided to a digital input of processing unit 170, the form output by sensor interface 160 may be digital. These examples are not limiting, as a digital input of processing unit 170 may be capable of coupling to an analog signal (e.g., detecting when the signal crosses thresholds), and an analog input of processing unit 170 may be capable of receiving a signal in digital form (e.g., if the digital signal is provided at a frequency within the constraints imposed by the response time of the analog input). In one or more embodiments, sensor interface 160 includes a serial or parallel data interface, through which information is provided according to a proprietary or industry protocol. In one or more embodiments, sensor interface 160 includes one or more filters in hardware or software, such as a low pass, high pass, or bandpass filter, an integrator or other smoothing filter, a decimation filter, an offset adjustment filter, and so forth.

Processing unit 170 is a computing device that receives the information from sensor interface 160 and processes the information. In one or more embodiments, information is fully processed in processing unit 170 to identify material types and to create a mapping of an irregularity. In other embodiments, information is partially processed in processing unit 170 and provided by processing unit 170 to another computing device (not shown) for further processing. For example, processing unit 170 may determine material properties from the information received from sensor interface 160, and provide the material properties externally to another computing device for identification of material types and mapping.

Processing unit 170 may further control position sensing device 150, such as to turn it on or off, set sensing parameters, or provide calibration settings.

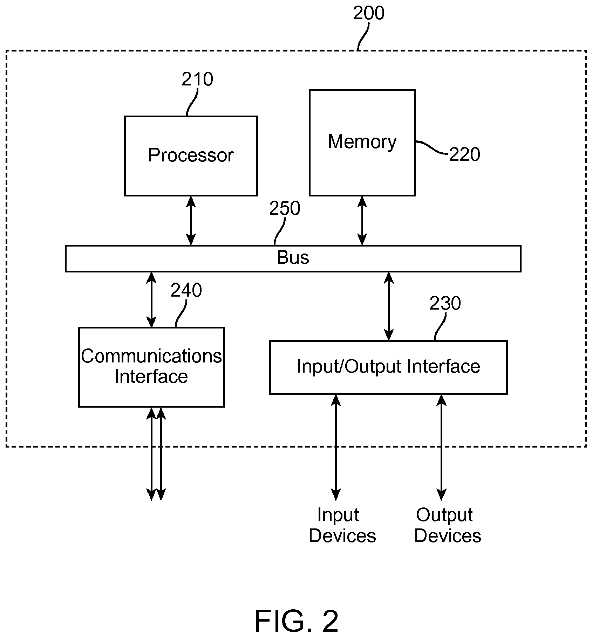

FIG. 2 illustrates an example of a computing device generally (e.g., processing unit 170). Computing device 200 includes a processor 210, a memory 220, an input/output interface 230, and a communication interface 240. A bus 250 provides a communication path between two or more of the components of computing device 200. The components shown are provided by way of illustration and are not limiting. Computing device 200 may have additional or fewer components, or multiple of the same component.

Processor 210 represents one or more of a general-purpose processor, digital signal processor, microprocessor, microcontroller, application specific integrated circuit (ASIC), field programmable gate array (FPGA), other circuitry effecting processor functionality, or a combination thereof, along with associated logic and interface circuitry.

Memory 220 represents one or both of volatile and non-volatile memory for storing information (e.g., instructions and data). Examples of memory include semiconductor memory devices such as EPROM, EEPROM, flash memory, RAM, or ROM devices, magnetic media such as internal hard disks or removable disks or magnetic tape, magneto-optical disks, CD-ROM and DVD-ROM disks, holographic disks, and the like.

Portions of FNE system 100 may be implemented as computer-readable instructions in memory 220 of computing device 200, executed by processor 210.

Input/output interface 230 represents electrical components and optional code that together provide an interface from the internal components of computing device 200 to external components. Examples include a driver integrated circuit with associated programming, or an interface to an external memory.

Communication interface 240 represents electrical components and optional code that together provides an interface from the internal components of computing device 200 to external networks, such as a network through which processor 210 provides information external to FNE system 100. Communication interface 240 may be bidirectional, such that, for example, data may be sent from computing device 200, and instructions and updates may be received by computing device 200.

Bus 250 represents one or more interfaces between components within computing device 200. For example, bus 250 may include a dedicated connection between processor 210 and memory 220 as well as a shared connection between processor 210 and multiple other components of computing device 200.

An embodiment of the disclosure relates to a non-transitory computer-readable storage medium (e.g., a memory 220) having computer code thereon for performing various computer-implemented operations. The term "computer-readable storage medium" is used herein to include any medium that is capable of storing or encoding a sequence of instructions or computer codes for performing the operations, methodologies, and techniques described herein. The media and computer code may be those specially designed and constructed for the purposes of the embodiments of the disclosure, or they may be of the kind well known and available to those having skill in the computer software arts.

Examples of computer code include machine code, such as produced by a compiler, and files containing higher-level code that are executed by a computer using an interpreter or a compiler. For example, an embodiment of the disclosure may be implemented using Java, C++, or other object-oriented programming language and development tools. Additional examples of computer code include encrypted code and compressed code. Moreover, an embodiment of the disclosure may be downloaded as a computer program product, which may be transferred from a remote computer (e.g., a server computer) to a requesting computer (e.g., a client computer or a different server computer) via a transmission channel. Another embodiment of the disclosure may be implemented in hardwired circuitry in place of, or in combination with, machine-executable software instructions.

The FNE system 100 allows a mapping of quantitative variations in material properties on insertion of probing element 120 into a material. In one or more embodiments, probing element 120 can be inserted into a patient (e.g., a human patient or other animal patient), a biological tissue or other material obtained from a patient, or other material. In one or more embodiments, force sensor 130 can be mounted relative to probing element 120, such that force sensor 130 remains partially or fully outside of the patient during insertion of the probing element 120. In one or more embodiments, FNE system 100 derives a mapping of spatial variations of one or more material properties of a material or a volume. In one or more embodiments, based on the mapping, FNE system 100 derives an indication of a likelihood of an irregularity in the material, such as caused by cancer or other disease. In the case of cancer, the FNE system 100 also can derive an indication of a grade or a stage of cancer in one or more embodiments.

Having described FNE system 100 in overview, some examples are next provided. The following examples describe specific aspects of some embodiments of this disclosure to illustrate and provide a description for those of ordinary skill in the art. The examples should not be construed as limiting this disclosure, as the examples merely provide specific methodology useful in understanding and practicing some embodiments of this disclosure.

Example 1

An FNE device for biomechanically determining local variations of mechanical tissue properties

Example 1 describes an FNE device, system and techniques for evaluation of a thyroid nodule. A difference in stiffness between healthy and diseased tissue is relatively common in thyroid cancers, and is typically initially investigated by palpation. Palpable thyroid nodules are found in 4-7% of the population, with thyroid carcinoma representing about 5-10% of these nodules.

Healthy and diseased tissues exhibit differences in mechanical properties, thus, the mechanical properties of tissue possess clinical diagnostic significance. Example 1 reports the design and development of an FNE device with an integrated FNAC needle that allows for quantitative and sensitive assessment of tissues and materials based on local variations in elastic, friction, and cutting forces upon needle insertion. A piezoelectric forcesensor mounted at the base of the FNA needle measures forces opposing needle penetration, with resolution in the $\mu$m scale.

Measurement precision ($\pm$ 5 $\mu$m) and axial resolution (about 20 $\mu$m) of a prototype FNE device were tested using control mm-sized gelatin matrices and an unripe pear, to asses needle penetration resistance, force heterogeneity and optimization of needle penetration velocity. The gelatin and pear have an analogous feel to healthy tissue and tumor tissue, respectively.

The FNE device was also demonstrated in quantitative, biomechanical differentiation of simulated thyroid tumor nodules in an ultrasound neck phantom, where fluid and solid nodules were probed in the phantom, coupled with ultrasound guidance. The data shows significantly higher force variations in solid nodules compared either to fluid nodules or to regions corresponding to healthy thyroid tissue within the ultrasound phantom. For example, with respect to 1-D force roughness, Ra = 6.5 mN, Rq = 8.25 mN; and with respect to biomechanical roughness, Ra = 0.0274 kN/m, Rq = 0.0395 kN/m. The results indicate future applications of the FNE device, system, and techniques for *in vivo* FNE biopsies based on force heterogeneity, to diagnose benign and malignant nodules in thyroid cancer. Thus, the FNE device, system, and techniques promise to be a primary diagnostic tool in the management of thyroid cancers, without the need for ultrasound instrumentation or access to a qualified pathologist for FNAC. Alternatively, the FNE device, system, and techniques could be used for ancillary diagnostics.

Palpation is the initial screening for thyroid cancer. On finding a solid nodule by palpation, a clinician generally refers the patient for a second level of screening, which is an ultrasound of the thyroid. Ultrasonography can be used to determine the presence of nonpalpable nodules as small as 1 mm within the thyroid tissue. Ultrasound is a mechanical imaging mode, as reflections from tissue occur where there are significant variations in density or elastic modulus. Sonographers may document the presence of calcification in thyroid nodules, because these hard, dense localizations within a solid nodule are correlated with various carcinomas of the thyroid. If nodules are indicated by the ultrasonography (as interpreted by the sonographer), a patient may be referred for a third level of screening, an ultrasound-assisted biopsy by FNAC. In this procedure, a hollow needle with a concentric stylette is inserted into the nodule several times, while drawing back the stylette so that the suction induced by the stylette draws a small sample of cells into the needle. The aspirated cells are usually smeared onto a microscope slide in preparation for subsequent cytological evaluation and diagnosis based on cell morphology.

The concepts of Example 1 allow progression from initial screening to diagnosis without ultrasound equipment, subjective evaluations of ultrasound images, or analysis of aspirated cells by a pathologist. Further, the resolution of the FNE device, system, and techniques surpasses the resolution achievable by ultrasound. A quantitative analysis of forces exerted on the needle of the FNE device of Example 1 as it traverses a material can discriminate between healthy and malignant tissues. Thus, the concepts of the Example 1 may be used alone, or to enhance the sensitivity and specificity of existing techniques.

When a needle enters tissue, the process follows a distinctive pattern. First, the needle pushes the tissue, increasing the insertion force steadily. During this phase, the tissue deforms until a stress limit is reached. Then, the needle punctures the tissue, followed by tissue relaxation, at which point the needle advances into the tissue. A puncture is observed as a sharp drop in insertion force. There are three main forces acting on a needle inserted into a soft tissue; namely, stiffness force, frictional force, and cutting force (at the tip of the needle). The total force for needle insertion depends predominantly on the local biomechanical properties of the tissue. Thus, the haptic perception due to this total force is correlated with the local biomechanical properties of the tissue. There can be significant differences in haptic perception on insertion of a needle into healthy tissue versus malignant thyroid tumors. For example, the haptic perception of insertion of an FNA needle into healthy tissues is similar to inserting a needle into gelatin, whereas the haptic perception for malignant tumors is similar to inserting a needle into an unripe pear.

There is significant variation in mechanical properties of materials at the tens of $\mu$m scale, below the resolution of ultrasound. At the cellular level, cancer cells are more compliant than healthy tissue; however, tumors are typically less compliant than healthy tissue. This is consistent with an increase in matrix deposition and cross-linking observed during cancer progression. The mechanical response of tissue is dominated by stiff structural elements in the peripheral stroma (e.g., collagen). Conceptually, as a needle tip passes through a region of healthy cells, measured elastic forces will be smaller than for healthy tissue, whereas the needle measures significantly higher forces than for healthy tissue on passing through peripheral stroma. Additionally, normal glandular epithelium and benign solid lesions exhibit a unimodal but distinct stiffness distribution. Malignancies, on the other hand, can display mechanical heterogeneity in line with histological appearance, and a characteristic lower stiffness peak in areas with densely packed tumor cells and little intervening stroma. The net result is a significant increase in the heterogeneity of local forces on the tens of $\mu$m size scale as the needle passes through a malignant nodule.

Efforts have been made to extend the capability of ultrasound to measure quantitative elastic properties *in vivo* in an approach called elastography (in this case, ultrasound elastography, or UE). In UE, elastic deformation is measured via ultrasound, and the resulting deformations are used to compute a local stiffness of the tissue at the resolution of medical ultrasound (0.2 to 0.3 mm at 5-12 MHz). At this size scale, healthy tissues exhibit lower stiffness than tumors. However, since UE measures deformation via ultrasonography (size scale in mm), it is unable to detect the heterogeneity that is present on a size scale in the tens of $\mu$m.

In another direction, AFM has shown capabilities in probing viscoelastic properties of cells and spatial mapping of cell mechanical properties, and has been explored for diagnostic applications. However, AFM is a surface technique, unable to probe subcutaneous tissue *in vivo* (such as thyroid nodules in a patient).

Example 1 sets forth an investigation for detection of heterogeneity, at depths useful for detection of properties of materials *in vivo,* with a size scale capability in the tens of $\mu$m. In general for one or more embodiments, depth range, such as associated with a distance at which a needle (or other probing element) can be inserted into a biological tissue, can correspond to a length of the needle and can be greater than about 600 $\mu$m, such as about 1 mm or greater, about 5 mm or greater, about 1 cm or greater, or about 2 cm or greater, and up to about 5 cm or greater, and spatial resolution can be less than about 1 mm, such as about 0.5 mm or less, about 0.3 mm or less, about 0.2 mm or less, about 100 $\mu$m or less, about 80 $\mu$m or less, or about 40 $\mu$m or less, and down to about 10 $\mu$m or less. The investigation involved mapping insertion forces, for quantitative assessment of insertion forces and tissue heterogeneity. Thus, a diagnostic technology is introduced, including a diagnostic technology for *in vivo* cancer diagnosis of solid tumors such as thyroid, breast and liver.

An FNE device, with a calibrated force sensor at the base of an FNA needle, was developed in the investigation.

The approach was validated using two techniques. In a first technique, measurement precision and resolution of the device were initially determined with an analogous specimens of mm scale gelatin matrices to represent healthy tissue and an unripe pear embedded within gelatin to represent a tumor in otherwise healthy tissue.

In a second technique, an ultrasound neck phantom was used to test the FNE device for biomechanical differentiation of different types of thyroid nodules. A portable ultrasound equipment was used for guidance of the FNE device. Simulated fluid and solid nodules in the ultrasound phantom were probed with the FNE device. The results show significantly higher force variations with distance (1-D Force Roughness: Ra = 6.5 mN, Rq = 8.25 mN) and biomechanical roughness (Ra = 0.0274 kN/m, Rq = 0.0395 kN/m) parameters in solid nodules as compared either to fluid nodules or to regions within the phantom corresponding to healthy thyroid tissue. The results were achieved at the micron-scale level.

The FNE device

FIGs. 3A-3C illustrate the FNE device of Example 1, where FIG. 3B is an expanded diagram of certain components of the device of FIG. 3A, and FIG. 3C is a depiction of how the device of FIG. 3A may be held.

Illustrated in FIG. 3A is a typical 25 gauge FNA needle (Becton, Dickinson and Company, USA) interchangeably mounted onto the head of the FNE device using a standard luer connection. The needle is narrow (e.g., about 500 $\mu$m outer diameter) to reduce bleeding, and long enough to penetrate the full depth of the thyroid (e.g., about 51 mm). The choice of a standard needle in this example leverages its low cost and the interchangeability afforded by a well-established needle industry. Additionally, the high degree of uniformity with which the needles are manufactured leads to repeatable forces in this mode of operation.

As illustrated in FIG. 3B, a cylindrical piezoelectric transducer (PZT-5A, Boston Piezo-Optics Inc., USA) is mounted in the head of the FNE device of FIG. 3A. The piezoelectric tube has electroless nickel electrodes (inner and outer walls of the tube serves as output electrodes) with a theoretically calculated capacitance of 3117 pF ($\pm$ 20%). Due to its high rigidity, simplicity, and low cost, a piezoelectric crystal was selected as the force sensor. While it is typical to measure a voltage across a piezoelectric crystal, such signals can be highly influenced by the capacitance of the sensor, which can lead to both hysteresis and drift. Consequently it was chosen to operate by measuring the charge, q, which tends to have a linear relation with stress both in theory and practice. The charge generated at the piezoelectric crystal is proportional to the force (F) acting on the device, according to equation (1), where, for the PZT-5A, $d_{31}$ is the piezoelectric constant ($d_{31}$ = -171 $\times$ $10^{-12}$ C/N), $r_o$ is the outside radius of the cylinder ($r_o$ = 0.0625"), $r_i$ is the inside radius of the cylinder $(r_i$ = 0.0425"), and $h$ is the axial height of the cylinder ($h$ = 0.500").

$$q = d_{31}\frac{2\pi r_o h}{\pi(r_o^2 - r_i^2)}F \qquad (1)$$

An ideal conversion factor of -5.1 $\times$ $10^{-9}$ C/N was computed. In the FNE device of Example 1, electrode size was reduced to fit the FNE device, and epoxy used to mount the electrodes, reducing the actual conversion factor to -4.3 $\times$ $10^{-9}$ C/N.

The end of the transducer was secured to the head, which was in turn secured to a 3D printed holder as shown in FIG. 3B. The holder was designed using SolidWorks 2013 (Dassault Systèmes SolidWorks Corp, France) and 3D printed by Makerbot Replicator2™ Desktop 3D printer (MakerBot Industries, USA). Outputs of the transducer were connected to a Bayonet Neill-Concelman (BNC) cable using insulated copper wires attached with small amounts of silver conductive epoxy (Chemtronics Inc., USA). A 5 M$\Omega$ resistor was connected electrically in parallel across the transducer for a high output impedance.

An advantage of positioning the transducer at a fixed location in the housing is that the processing of the signals from the transducer is less complex and more accurate without the need for adjusting measurements based on distance from the needle, which would be the case if the transducer was not affixed in the housing.

A further advantage of positioning the transducer within the housing is that the needle can be a smaller diameter than would be the case if a sensing element were positioned at the tip of the needle.

Yet a further advantage of positioning the transducer within the housing is that the needle can be an industry-standard needle (e.g., mass-produced), thereby reducing cost and increasing availability and consistency.

Yet a further advantage of positioning the transducer within the housing is that the needle may be extended substantially along its length into a material, rather than being prevented from extension into the material, as would be the case if a sensor were mounted along the needle.

Experimental Setup

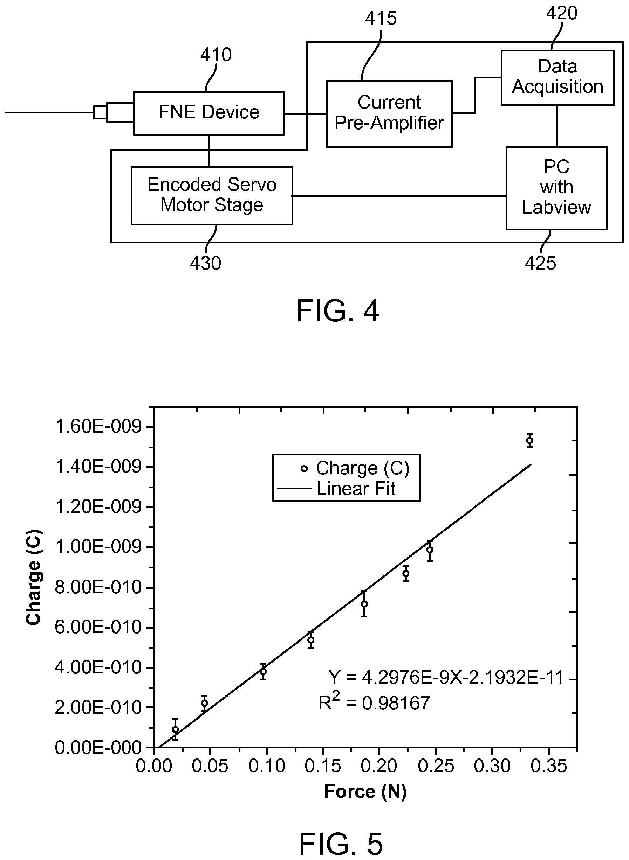

FIG. 4 illustrates the experimental setup for FNE measurements using an FNE device 410. While it was desired to measure charge directly, charge amplifiers tend to roll off at low frequencies. Consequently, it was chosen instead for Example 1 to measure and numerically integrate current over time to determine charge, and thereby improve performance of the system at low frequency.

Output of the FNE device 410 was connected to a low-noise current preamplifier 415 (SR570, Stanford Research Systems, USA). The output current signal was amplified at 10 nV/A gain with bandwidth 0.03 Hz-300 KHz. The amplified current signal was digitized using a data acquisition card 420 (NI USB 6259, National Instruments, USA). The digitized current signal was integrated with a custom written LabView 2013 (National Instruments, USA) program running on a dedicated computing device 425 (Intel Core 2, 6400 at 2.13 GHz, 2 GB RAM, 32 bit-MS Windows Vista Home Premium SP2) used for the data acquisition and position control of a single axis linear motion stage.

Linear motion of FNE device 410 was controlled by a single axis actuator stage 430 (LX26, Misumi Groups Inc., Suruga Seiki Co., Japan) with a manufacturer specified positioning repeatability of $\pm$ 5 $\mu$m and a maximum travel length of 200 mm. The stage was driven by a two-phase hybrid stepper motor (HT17-068, Applied Motion Products Inc., USA) controlled by a servomotor controller (AM3540i, Applied Motion Products Inc., USA). The positioning-accuracy of the linear stage in combination with the encoder was at least 10-12 $\mu$m.

Force Calibration: Gravitational force loading was chosen because of the elegant simplicity of the approach. Masses (m) between 0.5 g and 15 g were weighed using a digital balance, and the resulting gravitational force, $F_g$, was computed using $F_g = m\,g$, where $g = 9.81$ m/s$^2$ is the gravitational constant. FNE device 410 was mounted vertically and then smoothly loaded and unloaded with the calibrated masses. The change in piezoelectric charge from gravitational force loading was measured for each of the calibrated masses. FIG. 5 plots the measured force versus piezoelectric charge, where a first order polynomial (linear) fit slope of the calibration constant (N/C) is also shown. Table 2 shows the values that are plotted in FIG. 5.

**Table 2**

| Mass(g) | F=mg(N) | Current integral reading(A) | PreAmpGain(V/A) | Charge (C) |
|---|---|---|---|---|
| 1.923 | 0.01886463 | 0.0468 | 2.00E-09 | 9.36E-11 |
| 4.587 | 0.04499847 | 0.11 | 2.00E-09 | 2.20E-10 |
| 9.875 | 0.09687375 | 0.1888 | 2.00E-09 | 3.78E-10 |
| 14.183 | 0.13913523 | 0.2647 | 2.00E-09 | 5.29E-10 |
| 18.948 | 0.18587988 | 0.36125 | 2.00E-09 | 7.23E-10 |
| 22.725 | 0.22293225 | 0.435 | 2.00E-09 | 8.70E-10 |
| 24.926 | 0.24452406 | 0.4927 | 2.00E-09 | 9.85E-10 |
| 33.914 | 0.33269634 | 0.76525 | 2.00E-09 | 1.53E-09 |

Force calibration measurements were done at a preamp gain of 2 nA/V using a band pass filter of 0.03 Hz-100 kHz. After the calibration procedure, the calibrated masses were again loaded, and the force measured by FNE device 410 was verified to be in agreement with the force computed via $F_g = m\,g$.

Force Measurements: Force measurements were recorded at 1 kilosample (kS) per second while FNE device 410 was translated at 12 mm/s, providing a 12 $\mu$m per sample resolution. The following variables were stored on the computer: time stamp (s), stage position (mm), integrated current (A) and force (N). Typical measurement times were maintained below 8 s per trial.

Characterization of forces for needle insertion: Characterization of the forces during needle insertion into soft tissue is important for preoperative planning and realistic surgical simulations of many percutaneous therapies, including FNAC. There have been several theoretical models developed that describe needle-tissue interaction mechanics in terms of forces and displacement.

Conceptually, a needle inserted into a vacuum will experience inertial forces, $F_{inertial}(t)$. On insertion into materials, the material will resist penetration so that the needle experiences forces opposite the direction of penetration. These insertion forces are, in the order in which they are experienced, $F_{stiffness}(t)$, which describes forces that arise from the deformation of the tissue, experienced as an elastic restoring force at the tip of the needle; $F_{cutting}(t)$, which is due to cutting as the needle splits the tissue apart, experienced at and near the tip of the needle, and $F_{friction}(t)$, which describes friction as the needle wall slides past the tissue through which the needle has already penetrated, acting on the walls of the needle due to the relative motion between needle and tissue. Cutting forces include the plastic deformation from the act of cutting as well as the force resulting from tissue stiffness at the tip of the needle. Friction force is a function of the internal biomechanical properties (e.g., stiffness) of the tissue as well as the properties of the needle wall surface. Each of these forces can be expanded to describe higher order behavior as well. For instance, $F_{stiffness}(t)$, could also describe nonlinear stiffness or plastic deformation of the tissue.

A combined needle insertion force with respect to a given material is presented in equation (1).

$$F_{needle}(t) = F_{stiffness}(t) + F_{cutting}(t) + F_{friction}(t) + F_{inertial}(t) \qquad (1')$$

The measured total force is a function of the biomechanical properties of the respective tissue.

Results and Data Analysis - Analogous Specimen

Preliminary FNE force measurements were made using a specially prepared specimen of a cleaned 10 mm unripe pear slice embedded in 6% gelatin (gel). This specimen is analogous to a thyroid tumor surrounded by healthy tissue. Commercial unflavored gelatin powder (The Kroger Co., USA) was used to make the gel, with 1.2 g of dry gelatin powder dissolved in about 20 mL of boiling water. Red food coloring was added for clear differentiation from the pear layer. The gel and the pear were placed in a graduated type-1 glass vial (15 x 45 mm, Fisher Scientific International, Inc., USA) and allowed to solidify for 2 hours. Once the gel solidified, the sample was immediately used for FNE measurements under ambient conditions (at room temperature and pressure). Additional samples omitted the pear.

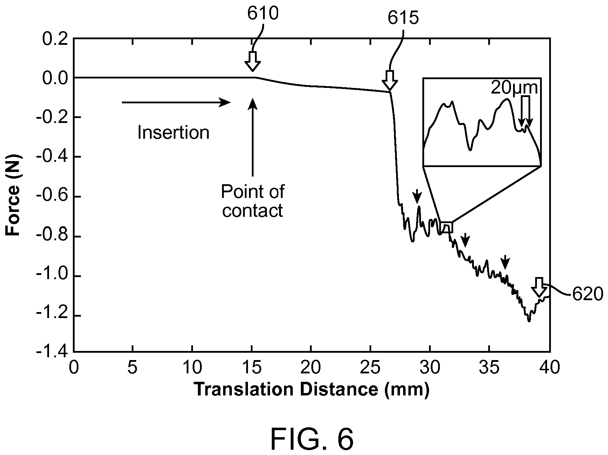

FIG. 6 is a plot of a typical force profile obtained as the FNE device 410 needle proceeded through air towards the contents of the glass vial, through the gel, and through the pear in the prepared specimen described above. Initially, as the needle traveled through air, approximately zero force is indicated. At a point 610 marked "Point of contact," the needle enters the gel. The force profile shows a slight slope as the needle traverses the gel, representing the resistive force acted on the needle by the gel. The smooth force profile for the gel implies that there is minimal variation in force, due to small variation in mechanical properties (e.g., it is homogeneous). As the needle next enters the pear at a point 615, the force profile shows significant heterogeneity as compared to the force profiles in air and gel, as indicated by small arrows on the force profile between point 615 and a point 625. A zoomed in portion of the force profile as the needle is within the pear layer is shown on the inset of FIG. 6, where it can be seen that features with different mechanical properties can be identified below 20 $\mu$m resolution.

In practice, the original absolute position of the tissue could slightly change due to tissue deformations occurring during needle insertion. Considering that the deformations within the tissue are small, F(t) may be converted to F(x) using the trajectory x(t), which allows plotting force F(x) versus distance d(x) profiles.

The measured current signal represents a charge generated at the device according to i = dq/dt, where i is the current and dq/dt is the change in charge with respect to time. The change in charge is proportional to a change in force, according to dq/dt $\propto$ $d_{31}$ (dF/dt). And dF/dx = dF/dt * dt/dx results in i $\propto$ $d_{31}$v dF/dx, where dF/dx has the units of stiffness (N/m), $d_{31}$ is the piezoelectric constant, v=dx/dt is the velocity, and i is the measured current. Because the piezoelectric constant and the velocity can be considered to remain constant throughout a measurement, the current signal is proportional to the stiffness (dF/dx), with some contributions from time-dependent force variations (dF/dt).

In an attempt to quantitatively differentiate the heterogeneity of local biomechanical properties, force analogs of 1-dimensional roughness parameters are used, which are referred to as force heterogeneity ($H_F$) and stiffness heterogeneity ($H_S$). The $H_F$ and $H_S$ one-dimensional parameters were calculated using Matlab, for force (F(x)) as per equations (2) and (3) and for the first derivative of force (dF/dx) as per equations (2') and (3').

*Average force heterogenally:*

$$H_{F,a} = \frac{1}{N} \sum_{j=1}^{N} |F_j - \bar{F}| \qquad (2)$$

*Average stiffness heterogeneity:*

$$H_{S,a} = \frac{1}{N} \sum_{j=1}^{N} \left| \left[\frac{dF}{dx}\right]_j - \overline{\left[\frac{dF}{dx}\right]} \right| \qquad (2')$$

*$\bar{F}$- Mean of force*

$$\overline{\left[\frac{dF}{dx}\right]}$$

*- Mean of first derivative of force*

*N - Number of data points*

*Roll mean square forces heterogeneity:*

$$H_{F,q} = \sqrt{\frac{1}{N}\sum_{j=1}^{N}\left|F_j - \overline{F}\right|^2} \qquad\qquad (3)$$

*Root mean equars stiffness heterogeneity:*

$$H_{S,q} = \sqrt{\frac{1}{N}\sum_{j=1}^{N}\left|\left[\frac{dF}{dx}\right]_j - \left[\frac{dF}{dx}\right]\right|^2} \qquad\qquad (3')$$

The calculated average force heterogeneity ($H_{F,a}$) and root mean square force heterogeneity ($H_{F,q}$) parameters are in units of mN. The stiffness heterogeneity ($H_{S,a}$) and root mean square stiffness heterogeneity ($H_{S,q}$) are in units of kN/m. As shown in equation (2), one-dimensional average force heterogeneity $H_{F,a}$ is an average deviation of all points of a force profile from a mean line over the evaluation length (according to the standards: ASME B46.1-1995, ASME B46.1-1985, ISO 4287-1997, ISO 4287/1-1997). As shown in equation (3), root mean square force heterogeneity $H_{F,q}$ is a root mean square value for an average of the measured deviations taken within the evaluation length and measured from the mean line.

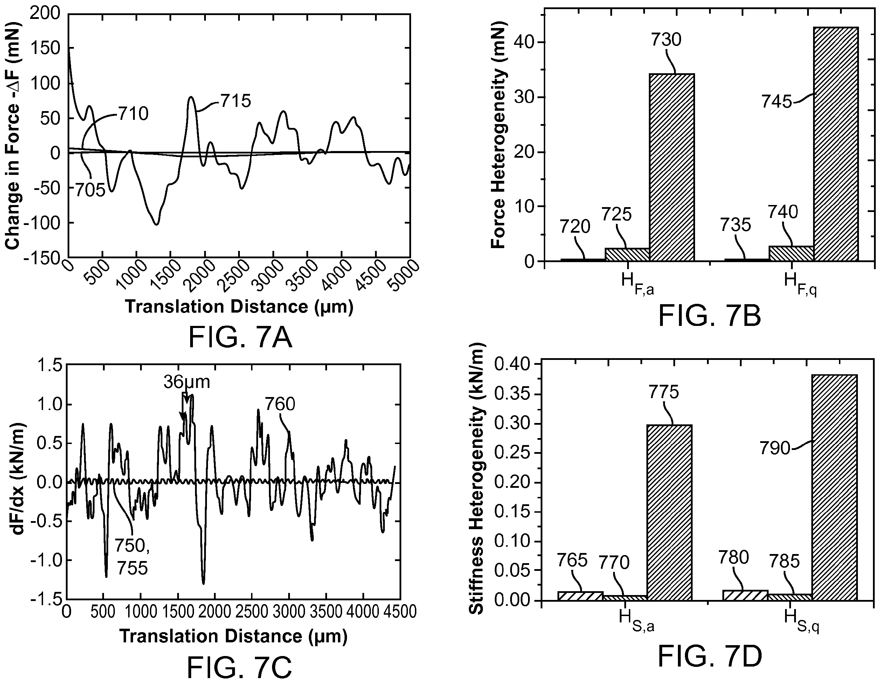

Raw data from the experiment results plotted in FIG. 6 were processed using a smoothing function in Matlab, with a five point moving average filter. FIG. 7A plots smoothed data as force versus distance profiles (with baseline adjustment using a first order polynomial fit) for air (curve 705), gel (curve 710), and pear (curve 715) data. Additionally a first derivative of force with respect to distance was calculated using a custom written code in Matlab, where the code calculates a derivative between 3 data points. In FIG. 7A, significantly high variations of mechanical properties within the pear layer region are clearly evident compared to the air region and the gel layer. FIG. 7B shows the force heterogeneity ($H_{F,a}$, $H_{F,q}$) parameters calculated for the force profiles shown in FIG. 7A. Bars 720, 735 represents the air, bars 725, 740 represent the gel, and bars 730, 745 represent the pair. FIG. 7B quantitatively describes the high variations of mechanical properties within the pear layer as compared to the air and gel regions. FIG. 7C shows a first derivative plot of a portion of the force profile of FIG. 7A. The first derivative of force with respect to distance has the same units as stiffness (N/m). As was evident from FIG. 7A (plots 705 and 710), the variation of force in the air and gel layers was minimal. Therefore, the first derivative plot of FIG. 7C shows that the derivative lines of air and gel (curves 750 and 755, respectively) overlap each other, whilst the pear region shows significantly high variations of the first derivative (curve 760). Quantification of these variations is shown by the stiffness heterogeneity parameters in FIG. 7D. High stiffness heterogeneity values ($H_{S,a}$, $H_{S,q}$) for the pear layer (bars 775, 790) represent the high local variations of mechanical properties within the pear layer, compared to the other regions (air, bars 765, 780 and gel, bars 770, 785).

The preliminary force measurements achieved by FNE device 410 for the analogous gel and pear specimen foresee promising results in the ability of FNE device 410 (and other embodiments of FNE device 110) in differentiating heterogeneity of tissue biomechanical properties to detect tumors and other irregularities.

Results and Data Analysis - Phantom

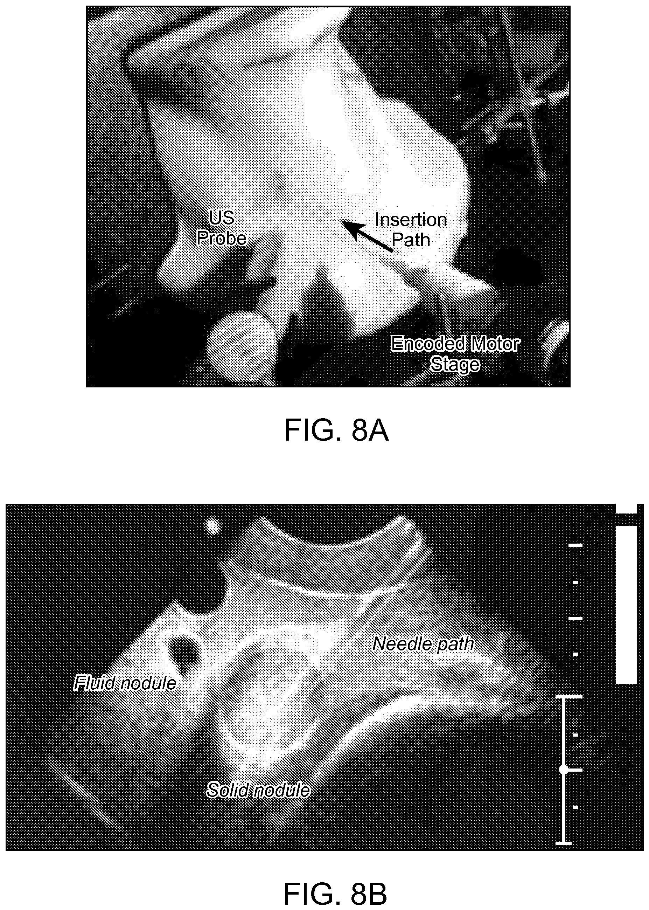

FIG. 8 provides an illustration of an anthropomorphic neck phantom (CIRS-074 thyroid ultrasound training phantom, Computerized Imaging Reference Systems, Inc., USA) used for further evaluation of the feasibility of FNE device 410 in thyroid tumor characterization and diagnosis. FIG. 8A provides an illustration of the phantom and FIG. 8B provides an ultrasound image of the phantom. The anthropomorphic neck (phantom) is a specially designed training tool and practice medium for ultrasound guided thyroid biopsy procedures for medical residents. The phantom contains a thyroid gland positioned within the anthropomorphic neck. Thyroid lobes contain two solid nodules (isoechoic stiff lesions) of diameter about 10-12 mm and four fluid nodules (cysts) of diameter about 8 mm. The phantom is made of Zerdine® encased in proprietary elastomer. The elastomer material is produced by polymerization of acrylamide with N,N'-methylene-bis-acrylamide in vacuumdegassed liquid solutions. The variation of local ultrasound reflections within the material is achieved by inclusion of alumina, boron nitride, and glass microsphere particles in different concentrations to make a permanent suspension of solid and liquid particles. Even though the material doesn't look exactly like thyroid tissue on the scale of tens of microns, the phantom is a close approximation, and provides a model material where the local mechanical properties vary over the size scale of the thyroid (centimeters).

Insertions of FNE device 410 into the phantom neck were done under real-time ultrasound guidance (using SonoSite 180plus ultrasound system, SonoSite Inc., USA). Reflections in an ultrasound image represent the local variations of acoustic impedance (Z) of the material. Acoustic impedance (Z) is a function of the density and Young's modulus of a material. Since the Young's modulus is a measure of the stiffness of a material, the contrast in heterogeneity seen on a sonogram represents local variations in biomechanical properties within the material. Therefore, this ultrasound phantom serves as a good specimen to test local variations of mechanical property heterogeneity to evaluate FNE device 410.

For a typical force measurement using FNE device 410, the insertion angle (typically $90^0 \pm 10^0$) was determined by manually inserting a needle alone, while observing the respective nodule on the ultrasound screen. Then, FNE device 410 was aligned on an insertion path (as shown in FIG. 8A) determined from the pre-determined insertion angle. An ultrasound probe (C11/7-4 Curved-Array Ultrasound Transducer, SonoSite Inc., USA, labeled "US Probe" in FIG. 8A) was fixed at a desired orientation using a holder to maintain a clear view of the insertion path. For each force measurement, the sonogram screen was recorded using an HD video camera (Canon T3i, 18MP, at 1920 x 1080 at 30 frames per second (fps)). FIG. 8B shows an example of an ultrasound image recorded during measurements. It was verified that applied pressure from the ultrasound probe on the phantom neck specimen was minimal so that it did not significantly affect the force measurements. A constant insertion speed of 12 mm/s was used in the measurements. This value was mainly based on the typical insertion speed observed during an FNAC biopsy.

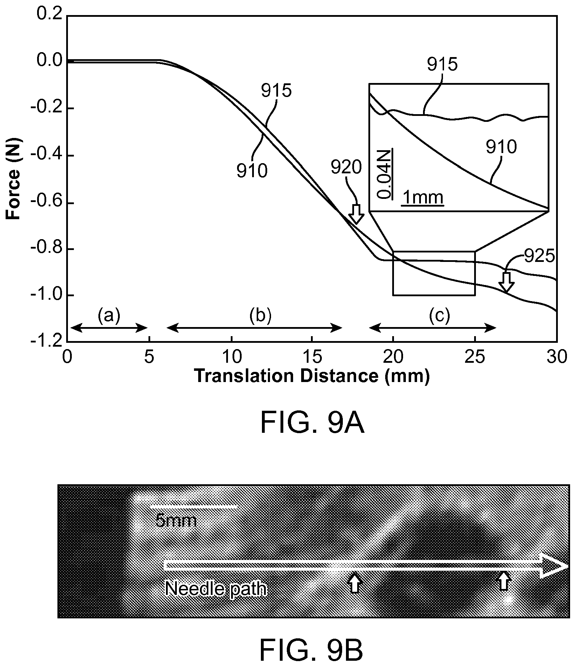

FIG. 9A shows a representative force profile for the FNE device 410 needle traversing through a fluid nodule within the phantom neck (curve 910), in comparison with a control (simulated healthy thyroid tissue, curve 915). The inset shows an expanded version of a portion of the force profiles. FIG. 9B shows an ultrasound image of the fluid nodule within the phantom neck, with the insertion path ("Needle Path") indicated. The fluid nodule (cyst) has a diameter of about 8 mm. The force curve (FIG. 9A) is composed of three main regions of the needle path. A region (a) (at the beginning of the insertion path) shows approximately zero force as the needle travels through air. The needle punctures through the phantom, and in region (b) (beginning slightly past 5 mm along the insertion path) there is a constant slope region corresponding to travel through artificial skin and then soft-tissue material. The needle then enters the fluid nodule at the leftmost dark arrow 920 of FIG. 9A (and leftmost dark arrow of FIG. 9B), and traverses the fluid nodule in region (c) of FIG. 9A before exiting the fluid nodule (rightmost dark arrow 925 of FIG. 9A and rightmost dark arrow of FIG. 9B). The small change of the slope in region (c) as compared to region (b) is due to relatively low resistance of the fluid in the fluid nodule as compared to the resistance of the artificial soft-tissue material. The smoothness of the curve in region (c) confirms low heterogeneity (high homogeneity) of the fluid, as well as easy penetration experienced by the FNE device 410 needle while traversing the fluid filled nodule.

Force profiles for FNE device 410 needle insertion through a 'no nodule region' of the phantom served as a control, and showed significant amount of heterogeneity, as shown in FIG. 9A. This was likely due to the inhomogeneous nature of the phantom elastomer material in that region (which was also evident from other ultrasound images not shown).

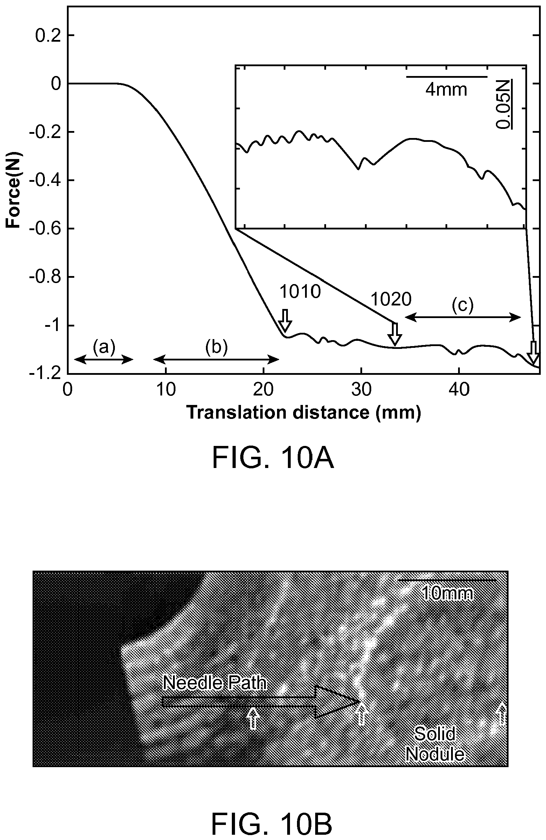

FIG. 10A shows a representative force profile for the FNE device 410 needle traversing through a solid nodule within the phantom neck. FIG. 10B shows an ultrasound image of the solid nodule within the phantom neck, with the insertion path ("Needle Path") indicated. The solid nodules in the phantom are designed to ultrasonically mimic the properties of actual thyroid carcinoma. The solid nodules have a diameter of about 15 mm. Similar to the fluid nodule force profile shown in FIG. 9A, approximately zero force is used in air (region (a)), and there is a constant slope for travel through the soft tissue (region (b)). The needle then enters an area with more heterogeneity (at about 22 mm, marked with a white arrow 1010 on FIG. 10A and the white arrow on FIG. 10B) and traverses to the solid nodule. The FNE needle punctures through the solid nodule (at about 34 mm, marked with a '*' at black arrow 1020 in FIG. 10A and at the black arrow in FIG. 10B) and travels through the solid nodule (region (c)). The force profile through region (c) shows substantially greater variations in force within the solid nodule region, as compared to the control and fluid filled nodule regions (FIG. 9). The inset in FIG. 10A shows a magnified section of region (c) in the inset.

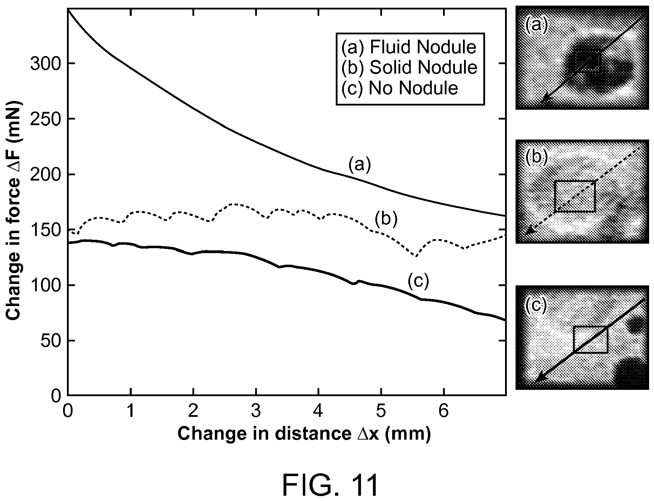

FIG. 11 shows differences in force profiles within the two kinds of nodule regions (liquid, solid) and a control region. FIG. 11 represents different portions of force profiles extracted from FIG. 9A and FIG. 10A, scaled together for side-by-side comparison. Corresponding ultrasound images are shown to the right, representing the FNE device 410 needle travel path for the respective curves. Clear differentiation of the solid nodule versus the fluid nodule was observed: the solid nodule force profile (b) shows significantly higher variations of force compared to the fluid nodule force profile (a). The control region force profile (c) also indicates force variations due to the inhomogeneous nature of the artificial soft tissue region.

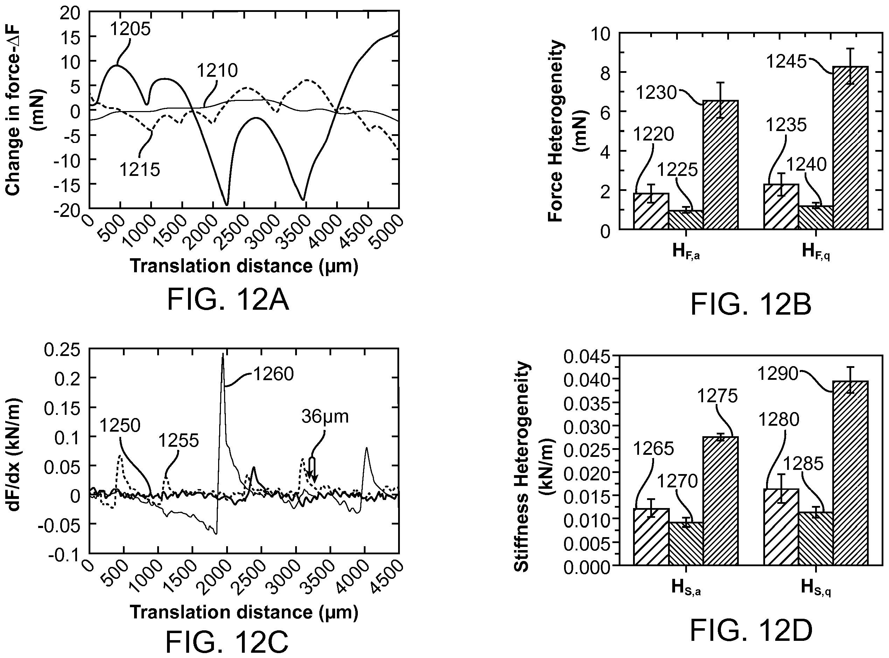

FIG. 12A shows a comparison of baseline adjusted force profiles ($\Delta F/dx$) for the solid nodule (curve 1205), fluid nodule (curve 1210) and control (curve 1215). The solid nodule force curve 1205 shows highest variations in force. Variations in force are due to the non-uniformity of mechanical properties within the solid nodule of the phantom, as was also seen in the sonogram of the phantom solid nodule in FIG. 10B.

FIG. 12B provides a quantification of force variations for the solid, fluid, and control curves of FIG. 12A. Force heterogeneity parameters $H_{F,a}$, $H_{F,q}$ calculated for the baseline adjusted force profiles of FIG. 12A are compared side-by-side in FIG. 12B. As can be seen in FIG. 12B, both average force heterogeneity $H_{F,a}$ and root mean square force heterogeneity $H_{F,q}$ values for the solid nodule (bars 1230, 1245) are several magnitudes higher than for the fluid nodule (bars 1225, 1240) and the control (bars 1220, 1235).

FIG. 12C shows a comparison of the first derivative of baseline adjusted force profiles (dF/dx) for the solid nodule (curve 1260), fluid nodule (curve 1250) and control (curve 1255). The first derivative of force with respect to translation distance shares the same units as one-dimensional tissue stiffness (N/m), and variation of dF/dx is proportional to the local variations of biomechanical properties of the material. The first derivative curve 1260 for the solid nodule shows significantly high variations, as compared to the fluid nodule and the control, due to the high heterogeneity of mechanical properties within the solid nodule. As can be seen from the indication of "36 $\mu$m" between two arrows in FIG. 12C, micrometer scale features with change in biomechanical properties can be resolved from the first derivative plot.

FIG. 12D provides a quantification of force variations for the solid, fluid, and control curves of FIG. 12C, Force heterogeneity parameters $H_{S,a}$, $H_{S,q}$ calculated for the baseline adjusted first derivative force profiles of FIG. 12C are compared side-by-side in FIG. 12D (solid nodule bars 1275, 1290, fluid nodule bars 1270, 1285 and control bars 1265, 1280). It is evident that the stiffness heterogeneity within the solid nodule is significantly higher as compared to the fluid nodule and the control, due to the significantly high local variations of mechanical properties within the solid nodule.

Accordingly, stiffness heterogeneity parameter values can be used in quantitative differentiation of solid or fluid nodules, including *in vivo* tumor nodules.

Discussion

Tumor development in healthy tissues is largely accompanied by complex microscopic structural changes in the extracellular matrix (ECM) and cellular architecture (about 1-10 $\mu$m), which can develop differentiable mechanical responses at the macroscopic tissue level (up to several hundred microns). Certain biomechanics characteristics of solid thyroid nodules have been associated with a higher likelihood of malignancy. Papillary carcinomas, the most common type of thyroid carcinomas, contain complex branching papillae, several hundred microns in size, that have a fibrovascular core covered by a single layer of tumor cells. Compared to the normal thyroid parenchyma, malignant nodules such as papillary thyroid carcinomas have been shown to display increased intranodular vascularity, irregular infiltrative margins, the presence of multiple microcalcifications, and a shape taller than the width measured in the transverse dimension. Because FNE device 410 can probe into the biomechanical heterogeneity of tumors within the papillae and calcified regions at greater resolution than achievable by other mechanical methods, quantitative FNE measurements are expected to be useful in predicting thyroid tumor behavior, in combination with other prognostic tumor markers.

Table 3 provides a comparison of techniques of measuring soft-tissue mechanical properties, including FNE. Like other mechanical imaging modalities, the FNE approach can be adapted for other palpable cancer lesions such as breast and liver lesions, independent of ultrasound.

Table 3

| Device | AFM | Ultrasound/ US Elastography | MRE (Magnetic Resonance Elastography) | FNE Device of Example 1 herein |
|---|---|---|---|---|
| Technique | Stiffness based on indentation of surface probe | Contrast in reflections of ultrasonic waves based on local variations of tissue acoustic impedance | Measures tissue mechanical properties by imaging *in vivo* shear wave propagation using MRI | Force measurement during needle insertion using a piezoelectric transducer, axially coupled with a biopsy needle |
| Measurement Range | 1-10 nm | 1 mm-10 cm | mm-1 m | 10 $\mu$m - 50 cm |
| Resolution | 1 nm-100 $\mu$m | 100 - 300 $\mu$m | mm | 10 - 20 $\mu$m |
| *In vivo/in vitro* | *In vitro* | *In vivo* | *In vivo* | *In vivo* |
| Cost | Highly expensive | Expensive | Expensive | Less expensive |
| Advantages | Cellular to tissue level information | Semi-Qualitative | Quantitative | Quantification of heterogeneity using roughness parameter values |

(continued)

| Device | AFM | Ultrasound/ US Elastography | MRE (Magnetic Resonance Elastography) | FNE Device of Example 1 herein |
|---|---|---|---|---|
| **Limitations** | Surface measurement, only *in vitro* | Limited depth capability | Shear wave propagation not always homogenous | No lateral force measurement in this example, but can be added in other implementations |

Example 1 demonstrated the resolution and measurement precision of the FNE device 410 in distinguishing the biomechanical properties and heterogeneity profiles between a gelatin matrix (homogeneous) and an unripe pear (heterogeneous), as well as between a solid (heterogeneous) and a fluid nodule (homogeneous) in a phantom neck model. To further evaluate the diagnostic significance of measuring biomechanical property heterogeneity at the cellular and tissue level, testing can be made *in vitro* for excised thyroid tissue patient samples. A database for stiffness and heterogeneity profiles from surgically removed fresh benign and malignant thyroid tumor patient samples can be generated to correlate tumor FNE data with tumor stage and grade cytopathology. Subsequent *in vivo* testing on human thyroid nodules can be used to study the operating parameters under practical conditions of an FNAC procedure, and further improve and develop the FNE device 410. It should be noted that a solid tumor can be benign (e.g., adenomatoid nodule or follicular adenoma) or malignant (papillary thyroid carcinoma, follicular carcinoma, and so forth), and a fluid-containing lesion may not be benign (e.g., cystic papillary thyroid carcinoma). Thus, further studies include assessment of tumors, lesions, other irregularities, margins of tumor resection or excision, and healthy tissue *in vivo* and *in vitro* to characterize forces expected by FNE device 410 in actual patients. Note that tissue deformation was assumed to be a comparatively small contributor to overall force and was thus not considered in detail in Example 1; however, there may be varying amounts of tissue deformations, which can be included in a comprehensive force model in a further improvement to the approach.

A hand-held portable device for combined FNAC and FNE is envisioned in the present disclosure, which can be modified for other *in vivo* percutaneous diagnosis and treatment methodologies.

Example 2

Another FNE device

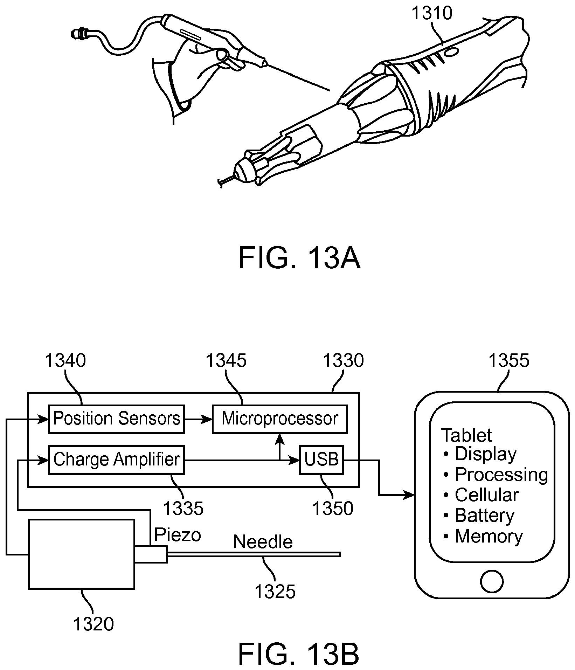

FIG. 13A illustrates another prototype of an FNE device 1310, along with an illustration of how it may be held. FIG. 13B illustrates a block diagram representing FNE device 1310. As shown in FIG. 13B, FNE device 1310 includes a piezoelectric force sensor 1320 in the form of a cylinder, and a 25-gauge FNA needle 1325 mounted to a handheld tool through the cylinder of force sensor 1320. FNE device 1310 is used to measure needle insertion forces quantitatively based primarily on tissue homogeneity, to detect benign versus malignant thyroid nodules. Also shown in FIG. 13B are components 1330, which may be incorporated within a housing of FNE device 1310, or connected to FNE device 1310, such as through wires. Components 1330 include a charge amplifier 1335, position sensor(s) 1340, a microprocessor 1345, and a universal serial bus (USB) interface 1350. USB interface 1350 is connected to a computing device 1355, shown as a tablet computer. USB interface 1350 may be wired or wireless. In other embodiments, USB interface 1350 is replaced with an alternative parallel or serial wired or wireless interface.

Charge amplifier 1335 generates a voltage proportional to insertion force. This voltage, together with data from position sensor(s) 1340, was collected by microprocessor 1345 (a computing device) and routed to secure computing device 1355 that provides data processing, data display, data storage, battery power and cellular communication.

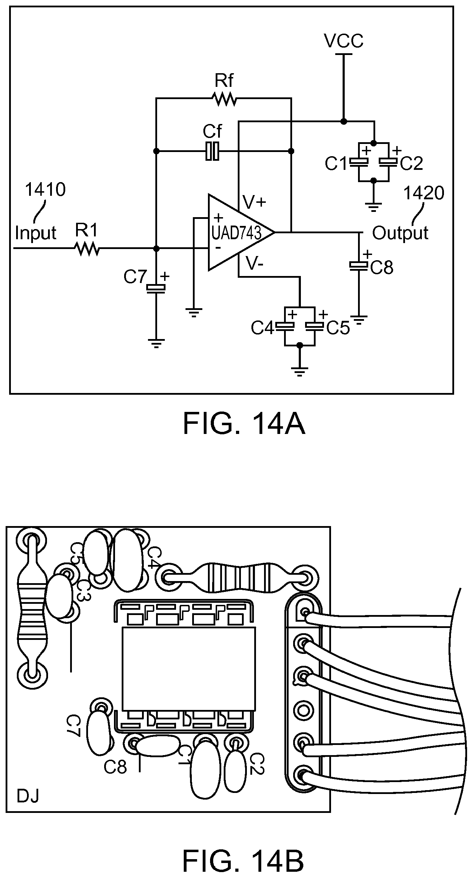

FIG. 14A is a schematic representing charge amplifier 1335 for FNE device 1310. Piezoelectric ceramics and crystals exhibit charge displacement in response to applied pressure. An indication of pressure applied to piezoelectric force sensor 1320 is received at charge amplifier 1335 at an input 1410. Charge amplifier 1335 outputs a voltage at an output 1420 that is proportional to the indication of the pressure applied to piezoelectric force sensor 1320 received at input 1410. The voltage is directly related to the insertion force on FNA needle 1325. FIG. 14B is an image of a prototype of charge amplifier 1335.

The cylinder, or tube, of the piezoelectric force sensor 1320 is segmented into quadrants that allow the device to measure not only axial insertion forces, but lateral insertion forces as well, providing substantially instantaneous feedback to an operator if the needle is not being inserted straight. The information regarding later insertion forces may also allow software correction for needle position due to angled insertion, as well as compensation for needle flexure.

In comparison to FNAC, FNE can provide a real-time quantitative elastographic approach with positional pre-

cision of 40 μm or greater. The technology is cost effective (approximately 100-fold cheaper) than ultrasound, MRI, or computerized tomography (CT), yet ten times the resolution of ultrasound, MRI or CT. Moreover, FNE device 1310 is much smaller than ultrasound, MRI, or CT equipment. The proposed insertion force elastographic approach bridges size scales from the single cell level, to tissues, to the organ level. This combination of size scales make FNE an intriguing approach not only for low-cost diagnostics but also for elastographic studies in intermediate size scales where material properties vary greatly. The technique can be adapted for a variety of applications, such as to detect palpable cancer lesions (breast, liver) independent of ultrasound methods. Further, treatment decisions can be made during use of FNE device 1310 for identification of tumors or other irregularities, and treatment may be applied during use of FNE device 1310, by adding features to FNE device 1310.

Tumors exhibit inhomogeneous elastic behavior as a consequence of collagen in the stroma and frequent calcium deposits. Such regions vary by orders of magnitude in elastic behavior from healthy cells, and so can be detected, such as by diagnostic ultrasound imaging, and even may be qualitatively detected by a clinician's hand during FNA needle insertion. Since the elastic inhomogeneity of the tissue results in inhomogeneous needle insertion forces, the proposed approach seeks to map quantitative insertion forces spatially as a low-cost cancer diagnostic.

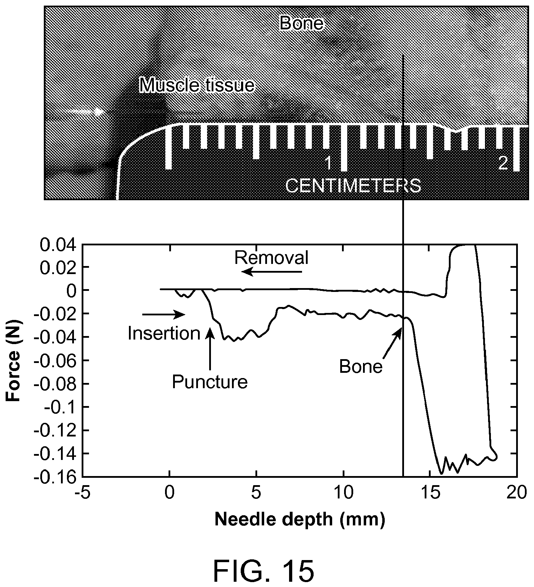

The force on the needle as it is inserted into biological tissue can be described as the sum of inertial, elastic, friction, and cutting forces. Typically, inertial forces are small since insertion takes place essentially at constant velocity, and they can be consequently neglected in most analyses. Elastic forces describe the restoring force that the tissue exerts on the needle in response to elastic deformation. Friction describes the resistance of the needle to velocity as it is inserted into the tissue, and is proportional both to velocity and to the surface area of the needle in contact with tissue. The cutting forces describe the act of splitting apart the tissue at the tip of the needle as it is driven deeper beneath the surface. Typical cutting models, describing needle penetration into homogeneous tissues, are characterized by a single elastic region, a subsequent puncture event, and then a friction and cutting region as the needle passes through the tissue. However, this model does not consider that materials may be inhomogeneous, and that multiple puncture events may occur when breaching skin or another material. For example, FIG. 15 shows a measured response of a needle penetrating into an inhomogeneous material of a bovine muscle, wherein the initial tissue puncture is followed by several smaller puncture events, as indicated by the undulations in force following the initial puncture. This series of features is absent when inserting a needle into a homogenous material. Consequently, FNE device 1310 provides a quantitative metric of tissue inhomogeneity.

Position of the needle can be mapped using resistive encoders (potentiometers), where the resistance is measured and converted to digital values in an ADC. (For example, many microcontrollers have integrated ADCs). Linear potentiometers with 4 cm travel can be digitized to 10-bit precision to achieve a positional precision of 40 μm, for example. This positional precision using low-cost components provides approximately ten times finer resolution than the diffraction limit of ultrasound, MRI or CT. Consequently, FNE device 1310 is a desirable approach to elastography. The proposed insertion force elastographic approach bridges size scales from the single cell level, to tissues, to the organ level, a capability not shared by ultrasound, MRI or CT.

If magnetic sensing used for needle positioning creates interference with optional ultrasound guidance, alternate sensing mechanisms can be used, such as a 6-axis gyroscope with 3-axis accelerometers, and sensing of needle insertion depth through changes of the second harmonic frequency of the needle, which depends on how far the needle extends into a tissue.

Example 3

Creation of a database

Tissue stiffness varies significantly between various portions of a human body. For example, soft tissue elasticity is less than 0.1 kPa for bone marrow, between 0.1 and 1 kPa for brain tissue, between 1 and 10 kPa for fat, around 10 kPa for muscle, and greater than 20 kPa for bone. Additionally, as described above, irregularities exhibit different tissue stiffness, and may change over time. For example, tumors change material properties as they progress.

The most reliable prognostic tumor marker currently being used clinically is tumor stage and grade (see, e.g., Table 4, for benign to malignant thyroid cancel nodules, where benign is scored as a '1'; stiffness is comparative to normal tissue).

**Table 4**

| 1 | 2 | 3 | 4 | 5 |
|---|---|---|---|---|
| Soft, homogeneous | Partly stiff, heterogeneous | Soft, homogeneous, peripheral stiff region | Stiff (homogeneous or otherwise) | Very stiff (may be heterogeneous), cysts may show distinct elastic region within stiff nodule |

Such grading, and subsequent diagnosis, is made by core biopsy or FNA of tumor mass, but grading and staging may not be accurate on these types of specimen. Cell stiffness is intimately related to cellular cytoskeletal remodeling. Cell stiffness plays an important role in tumor motility, invasion, and metastasis; however, tissue biomechanics and heterogeneity may be a more quantitative marker to predict tumor behavior.

A technique for correlating tumor stage and grade with tumor FNE measurements is next discussed.

Analysis using FNE can be made of surgically removed thyroid nodules from 50 patients, and the measurements correlated with Young's modulus (determined using AFM) and tumor histology type, grade, and stage of the resected tumor. Such an analysis would, for example, include tumors from patients between 18 and 80 years old, both female and male. (Thyroid cancer is rarely seen in patients less than 18 years old.) Alternatively, tumors could be characterized for more narrow age ranges, or individually for male and female, to identify distinctions based on age or sex. Analysis could be made across races or ethnicities, or individually for races or ethnicities, such as to identify whether there are distinctions based on race or ethnicity. To limit the number of variables, subjects with non-cancer severe medical conditions (such as hypertension, diabetes and bleeding problems) may be excluded.

To construct a database correlating tumor information with FNE data, identifying information (e.g., age, sex, race or ethnicity) of the patients from which the tumors are extracted is included in the database, along with tumor information (e.g., stage, grade, AFM measurements, and FNE measurements). For privacy concerns, each tumor may be identified by a number rather than by patient name.

Clinically applicable protocols can be used for obtaining and processing thyroid cancer tissue for the analyses. For example, measurements can be done on a fresh tissue sample within 1-2 hours of obtaining the sample. Twenty-five FNE measurements can be recorded for each tissue sample, in terms of force versus needle penetration depth as the needle is inserted into the tumor (e.g., under ultrasound guidance). The position of the needle, penetration depth, and angle of rotation can be controlled for 3D mapping using magnetic sensors. The data can be stored on a secure computing device for further analysis. Young's modulus of a sample can be derived based on the penetration distance of the needle within the tissue, and 3D maps can be generated of the tissue mechanics based on different orientations of the needle within the region of interest. Samples can be scored (e.g., as shown in Table 4), based on biomechanical patterns in a region of interest. For data fitting, peaks can be located using a peak analysis function, and a multi-peak fit can be applied to the stiffness distribution. Data can be recorded as a mean with standard deviation. The statistical significance of differences in mean values can be assessed using paired Student's t-test ($P \leq 0.05$).

For comparison purposes, five biopsies can be evaluated by standard pathological procedures for each thyroid tissue sample. For example, one biopsy can be used for AFM analysis directly after removal.

After FNE, all five biopsy samples can be retrieved, formalin-fixed and paraffin-embedded according to standard histological procedures. Sections with a thickness of about 5 $\mu$m can be cut and transferred onto coated glass slides. The first and last slides of sequential sections can be stained with hematoxylin and eosin (H&E) stain. Subsequent histopathological examination can include assessing the type of lesion (e.g., as per Table 4) and determining a number of standard histopathological markers (e.g., extent of tumor infiltration, fibrosis, necrosis and lymphocytic infiltration).

Biopsy specimens can be immediately transferred to ice-cold isotonic Ringer solution supplemented with glucose and a protease inhibitor and kept at 4 °C to minimize tissue degradation. Each specimen can be immobilized on a plastic dish with a thin layer of epoxy glue. AFM can be carried out at 37 °C (e.g., using a Catalyst AFM mounted on an inverted optical microscope). Up to 22 different $20 \times 20 \ \mu m^2$ force-volume (F-v) maps over $24 \times 24$ point grids (576 force-displacement curves per map and pixel size of 833 nm) can be recorded. F-v maps spaced at about 500 $\mu$m apart can be acquired systematically across the sample surface. Maximum applied loading force can be set to 1.0 nN, with indentation depths of about 150-3000 nm depending on intrinsic mechanical differences within each biopsy. Force curves can be analyzed using a Hertz-Sneddon model. The stiffness values (KPa) calculated from force curves can be spatially plotted to yield colorcoded stiffness maps (e.g., using MATLAB, Mathworks). Correlation analysis can be performed between FNE biomechanical/ heterogeneity profiles, AFM tissue stiffness measurements, and tumor pathological stage and grade.

A database created by the technique described can be used to assess the overall coherence between different histopathologies of thyroid cancer (or other irregularity).

The technique for creating a database described is presented by way of example and is not limiting. Additional tests may be performed, test conditions may be changed, the number of measurements and orientation angles can be increased for FNE measurements, additional correlations may be made, and so forth.

Example 4

An FNE device for *in vivo* measurement of breast tissue

FNAB (fine needle aspiration biopsy) has been used for diagnosis and screening because it is a rapid, cost-effective technique for minimally invasive breast lesion evaluation. However, FNAB-based diagnoses of breast cancer have largely been replaced by more invasive core biopsies, because interpretation of FNAB specimens lack architecture patterns, and FNAB requires evaluation by an experienced cytopathologist, and cannot distinguish between *in situ* and invasive carcinomas. Another technique, elastography, images the elastic properties of tissues, and has recently been used to determine relationships between different structures and their tissue elasticity, which can help diagnose malignant tumors. Healthy breast tissue is an inhomogeneous structure predominantly composed of fat and glandular tissues with distinct elastic properties, and there is a difference in elastic modulus (stiffness) between normal breast tissues and tumors. Malignant changes correlate with changes in tissue stiffness, resulting in extremely hard modulus. For example, studies suggest up to a fifteen-fold increase in the Young's modulus of infiltrating ductal carcinoma compared with normal breast tissue due to associated desmoplastic reaction, surrounding tissue infiltration and ductal involvement. By comparison, papillomas are about five-fold stiffer than normal tissue. Elastography-based stiffness imaging may provide additional improved sensitivity, objectivity or details, such as stiffness patterns, compared to manual palpation; however, elastography is expensive (e.g., greater than $200K), qualitative, and requires specialized instrumentation and operational skills. Additionally, breast elastography has limitations due to structural aspects of breast tissue and nodules, as well as lesion depth, and requires operator experience to obtain reproducible results. Thus there remains an unmet need for quantitative, cost-effective and easy-to-use breast cancer early diagnosis techniques.

By measuring *in vivo* tissue stiffness, breast cancer may be diagnosed, as tumors are stiffer than surrounding tissues and malignant tumors are much stiffer than benign tumors (and, paradoxically, malignant cells are much softer than normal cells).

Cytoskeletal actin is of importance in determining mechanical properties of cells. Using AFM, it was found that metastatic cells isolated from patients' pleural fluid were 70% softer than normal cells, suggesting metastasis might be promoted by cell compliance. At the macro level, spiculated tissue around cancer is resistant to deformation, thus feels hard when palpated. Therefore, tissue elasticity will differ depending on measurement scale. Biological tissue elasticity, which is anisotropic and nonlinear, will differ depending on direction and extent of deformation. Mechano-response of whole tumors is dominated by stiff structural elements in the peripheral stroma (e.g., collagen), increased matrix deposition and cross-linking during cancer progression. Normal glandular tissue, benign lesions and malignant tumors exhibit qualitatively unique biomechanical signatures, reproducible across different patients. Malignancies display mechanical heterogeneity in line with histological appearance and a characteristic lower stiffness peak in areas with densely packed tumor cells and little intervening stroma. Normal glandular epithelium and benign solid lesions, on the other hand, exhibit unimodal but distinct stiffness distribution. Breast cancer tissue shows higher elastic modulus (Young's modulus) than normal gland tissue. Overall, it is clear that nanomechanical profiling provides quantitative indicators in clinical diagnosis of palpable cancers, such as breast cancer, with translational significance.

Results from mechanical measurements of cancer cells and tissue substantiate using large-scale force mapping to improve sensitivity of breast cancer diagnosis.

Relationships between tumor hardness and cytological diagnoses were studied in an initial clinical study. Thyroid tumors (609 of them) were categorized into tumor or non-tumor based on *in vivo* stiffness testing of solid thyroid gland tumors by probing with hypodermic needles and manually detecting stiffness encountered by the needles. Stiffness rankings were then correlated to final cytological diagnoses. Cancer detection sensitivity of 0.81 and specificity of 0.89 was achieved. However, major limitations of this technique included qualitative assessment of thyroid nodule stiffness from resistance to needle penetration, which was subject to operator bias and lack of quality control. The FNE devices of the present disclosure (e.g., FNE devices 110, 410 and 1310) provide for improved sensitivity and specificity with quantitative assessment.

The FNE techniques described in this disclosure further provide for determining margins of irregularities. Finding margins of breast tumors, for example, remains a challenge. For example, in early stage breast cancer therapy, a tumor is located preoperatively by mammogram, ultrasound or MRI; then, during surgery (lumpectomy), the tumor mass is removed along with a buffer of normal tissue to avoid the necessity of subsequent surgery. Several studies have determined that margin status is one of the most important factors in predicting local recurrence. To verify that a buffer of normal tissue has been obtained, during surgery, excised tissue is inked and evaluated pathologically to assess margin as positive (cancer cells at/very close to inked surface), close (cancer cells within about 1-2 mm) or negative (cancer cells farther, typically greater than 1-2 mm). However, intra-operative pathological assessment, such as frozen section analysis (FSA) of breast fat tissue to obtain adequate surgical margins, is challenging. Besides FSA, other techniques have been tested with varying success to evaluate margins, including intraoperative ultrasound and imprint cytology (IC). However, these techniques are not used routinely due to technical and other limitations (see, e.g., Table 5, presenting challenges in the detection of tumor margin interface).

**Table 5**

| | Gross examination | Intra-operative ultrasound (US) | FSA | IC | FNE |
|---|---|---|---|---|---|
| Specimen/ preparation | Palpable mass: inked, sliced for pathology | Transducer used on excised tissue and remaining breast tissue | Palpable, nonpalpable cancers; tumors 2-3mm of inked margins | Tissue touched on slides, stained, screened for malignant cells | No preparation required |
| Margin Evaluation | Qualitative, pathology + palpation | Surgeon must be experienced in US | Measurement in mm range | Presence or absence of epithelial or atypical cells | Micron range stiffness |
| Efficacy | Not accurate | Useful for localization during surgery | Fairly reliable but false positive/ negative occur | Rapid (10min/ slide) Reliable with experienced interpretation | Portable, rapid, low cost, pre- or intraoperative |
| Limitations | Limited intraoperative value, microscopy of tumor cell difficult, slow | ~50% non-palpable lesions US visible, DCIS difficult to detect | Incomplete fat tissue, tumor-depth, limited use in small tumors; labor, cost intensive | Sample cells limited to excision surface, low sensitivity in lowgrade tumors | Tumor stiffness database desired |
| Pathology expertise | Needed | No | Needed | Needed | No |
| Time | 2 to 5 min | Variable | At least 15 min/ section | 5 to 10 min | Instant |

Accordingly, there remains a need for a combination of better preoperative and intraoperative approaches to complement pathologic assessment for obtaining adequate surgical margins. FNE is minimally invasive, and may be used pre- or intra-operatively to evaluate palpable or mammographic breast lesions based on differences in mechanical properties (Young's modulus) between healthy and cancerous tissues.

In this Example 4, a prototype FNE device is integrated with an FNA breast biopsy needle for 3D biomechanical mapping of suspected breast cancer nodules and analysis of Young's modulus and micron-scale nodule stiffness heterogeneity. The design of Example 4 includes a user interface with a quantitative mechanical data display for real-time evaluation, documentation and decision-making.

The FNE device includes a 25-gauge FNA needle mounted to a handheld tool through a force-sensing piezoelectric cylinder. A charge amplifier generates a voltage proportional to insertion force. That voltage, with data from a position sensor, is collected by a microprocessor and routed to a computing device that provides data processing, data display, data storage and communication. Due to the private nature of the data, the computing device may be secure, and the data storage may be secure.

The force-sensing piezoelectric cylinder includes a piezoelectric material that exhibits charge displacement in response to applied pressure. A charge amplifier produces a voltage output that is proportional to the pressure applied to the piezoelectric cylinder, such that the voltage output is a measurement of insertion force on the FNA needle. The FNE device piezoelectric tube measures axial insertion forces, and is segmented into quadrants that allow the device to also measure lateral insertion forces. This provides instantaneous feedback to the operator about needle insertion angle, and provides for software correction for needle position (e.g., due to insertion angle, or due to needle position changing as a consequence of needle flexure).

Needle position of the FNE device of Example 4 is mapped using resistive encoders (potentiometers). Many low-cost microcontrollers have on-board ADCs that can be used to digitize the potentiometer values. Linear potentiometers with 4 cm travel can be digitized to 10-bit precision to achieve a positional precision of 40 $\mu$m, which is approximately 10 times finer than the diffraction limit of ultrasound, MRI or CT.

Ultrasound is used for position sensing. Alternatively, a 6-axis gyroscope with 3-axis accelerometers may be used for position sensing, or position sensing may be based on changes of the second harmonic frequency of the needle, which depends on needle length inside the tissue.

# EP 3 179 921 B1

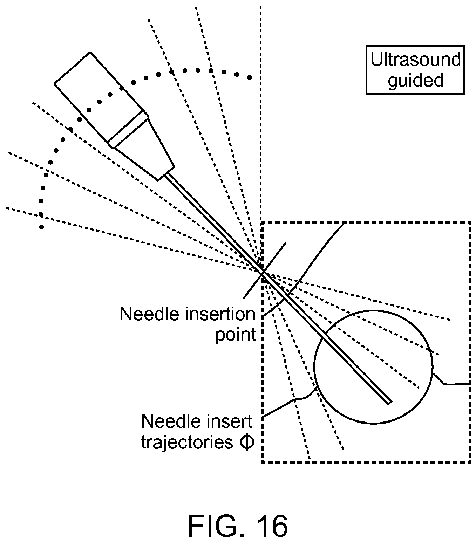

FIG. 16 illustrates mapping of an *in vivo* breast tumor using the FNE device of Example 4. As shown, the needle of the FNE device is inserted at the needle insertion point, then moved at different (3D) angles and depths to map the tumor from the force on the needle as detected by the force sensor.

A database of different breast cancer histological types and stages based on tumor heterogeneity and biomechanics is based on many samples of suspect breast nodules from patients undergoing partial or complete breast removal surgery. The database can be used to validate FNE diagnostic sensitivity and specificity, and to correlate with histopathology. Measurement protocols are described for *in vitro* FNE and AFM, including implementing blind studies. For the database, FNE is used to analyze resected breast nodules from 60 female patients between 18 and 80 years old, and correlate force measurements with Young's modulus (AFM) and tumor histology type, grade, and stage. The patients exclude subjects with non-cancer severe medical conditions such as uncontrolled hypertension, uncontrolled diabetes and severe bleeding dyscrasias. The techniques used for gathering the data for the database are as described with respect to Example 3.

Additionally, FNE data on all six margins of excised tissue (superior, inferior, medial, lateral, anterior, and posterior) is compared to gross pathological evaluations to determine sensitivity of FNE measurements in predicting clear margins.

Closing comments

Thus has been described in the present disclosure devices, systems, and techniques for minimally invasive *in vivo* FNE biopsies based on force heterogeneity. Advantages include reduced equipment size, reduced system cost, rapid diagnosis, and diagnosis in low-resource settings (e.g., unavailability of ultrasound instrumentation, or lack of access to a qualified FNAC pathologist). Additionally, the FNE devices, systems and techniques provide for an ancillary diagnostic tool. With respect to margins (e.g., in lumpectomies or other surgeries), the FNE devices, systems, and techniques can be used during surgery to define resection margins (e.g., rather than taking multiple frozen section examinations, which can be very inaccurate, time consuming (at least 15 minutes per section), and require pathology and histology support).

The FNE device described in the present disclosure may be implemented as an attachment to another device, such as a biopsy gun. In such an implementation, the FNE device may include a sensor and a wired or wireless communication interface for communicating externally. For example, referring to FIG. 1, FNE device 110 may include probing element 120, force sensor 130, and housing unit 140 as illustrated, and may further include a communication interface for providing data received from force sensor 130 to an external device such as computing device.

As used herein, the singular terms "a," "an," and "the" include plural referents unless the context clearly dictates otherwise. Thus, for example, reference to an object can include multiple objects unless the context clearly dictates otherwise.

As used herein, the terms "substantially," "approximately," and "about" are used to describe and account for small variations. When used in conjunction with an event or circumstance, the terms can refer to instances in which the event or circumstance occurs precisely as well as instances in which the event or circumstance occurs to a close approximation. For example, the terms can refer to less than or equal to $\pm 10\%$, such as less than or equal to $\pm 5\%$, less than or equal to $\pm 4\%$, less than or equal to $\pm 3\%$, less than or equal to $\pm 2\%$, less than or equal to $\pm 1\%$, less than or equal to $\pm 0.5\%$, less than or equal to $\pm 0.1\%$, or less than or equal to $\pm 0.05\%$.

As used herein, the terms "connect," "connected," and "connection" refer to an operational coupling or linking. Connected objects can be directly coupled to one another or can be indirectly coupled to one another, such as via another set of objects.

As used herein, the term "size" refers to a characteristic dimension of an object. Thus, for example, a size of an object that is spherical can refer to a diameter of the object. In the case of an object that is non-spherical, a size of the non-spherical object can refer to a diameter of a corresponding spherical object, where the corresponding spherical object exhibits or has a particular set of derivable or measurable properties that are substantially the same as those of the non-spherical object. When referring to a set of objects as having a particular size, it is contemplated that the objects can have a distribution of sizes around the particular size. Thus, as used herein, a size of a set of objects can refer to a typical size of a distribution of sizes, such as an average size, a median size, or a peak size.

While the disclosure has been described with reference to the specific embodiments thereof, it should be understood by those skilled in the art that various changes may be made without departing from the scope of the disclosure as defined by the appended claims.

**Claims**

1. An elastography system (100) comprising:

a hand-held elastography device (110) including:

a housing (140);
a probing element (120) removably attached to the housing; and
a force sensor (130) attached within the housing so as to have a fixed spatial relationship relative to the probing element and a fixed spatial relationship relative to the housing, the force sensor connected to the probing element; and

a position sensing device (150) connected to the elastography device;
wherein the force sensor is configured to produce a signal indicative of a force applied to the probing element upon insertion of the probing element into a material, and the elastography system further comprises a processing unit (170) connected to the force sensor, wherein the processing unit is configured to, based on changes in the signal, derive a mapping of spatial variations of a material property within the material in response to the variations of the material property during traversal of the probing element along an insertion path through the material.

2. The elastography system of claim 1, wherein the probing element is a needle, a tube, or a wire.

3. The elastography system of any of claims 1 or 2, wherein the force sensor is a piezoelectric crystal, a strain gauge, or a displacement sensor.

4. The elastography system of any of claims 1, 2 or 3, wherein the position sensing device is an encoded stage, or wherein the position sensing device is an ultrasound displacement sensor, a magnetic displacement sensor, or an electric displacement sensor.

5. The elastography system of any one of claims 1-4, wherein the material is a biological tissue, and the processing unit is configured to, based on the mapping, derive an indication of likelihood of an irregularity of the biological tissue, or wherein the material is a biological tissue, and the processing unit is configured to, based on the mapping, derive an indication of likelihood of cancer, or wherein the material is a biological tissue, and the processing unit is configured to, based on the mapping, derive an indication of a grade or a stage of cancer.

6. The elastography system of any of claims 1-5, wherein the probing element is one of a fine needle aspiration (FNA) needle or a fine needle biopsy (FNB) needle.

7. A hand-held elastrography system (100) comprising:
a housing (140);

a needle (120) removably attached to the housing (140);
a force sensor (130) positioned within the housing and fixedly mounted relative to the needle, the force sensor configured to detect a force applied to the needle; and
a processing unit (170) connected to the force sensor, wherein the processing unit is configured to derive a mapping of spatial variations of a material property within a biological tissue based on changes in the force detected at the force sensor, in response to the variations of the material property during a traversal of the needle along an insertion path through the biological tissue, and the processing unit is configured to, based on the mapping, derive an indication of likelihood of an irregularity of the biological tissue.

8. The elastography system of claim 7, further comprising a position sensing device attached to the housing, wherein the processing unit is coupled to the position sensing device and is configured to adjust a trajectory of the needle based on information from the position sensing device.

9. The elastography system of any of claims 7 or 8, wherein the force sensor is a piezoelectric device, further comprising:
a charge amplifier coupled to the force sensor to detect accumulated charge, the charge amplifier further coupled to the processing unit and outputting a voltage that is the parameter representative of the force detected at the force sensor.

10. The elastography system of any of claims 7, 8 or 9, further comprising:
a serial interface coupled to the processing unit and configured to communicate with devices external to the housing.

11. The elastography system of any of claims 7-10, wherein the needle is a fine needle aspiration (FNA) needle.

**12.** The elastography system of any of claims 7-11, wherein the force sensor is configured to detect axial and lateral force applied to the needle.

**Patentansprüche**

**1.** Elastographiesystem (100), umfassend:
eine tragbare Elastographievorrichtung (110) einschließlich:

eines Gehäuses (140);
eines Sondenelements (120), das entfernbar an dem Gehäuse angebracht ist; und
eines Kraftsensors (130), der innerhalb des Gehäuses angebracht ist, um eine feste räumliche Beziehung relativ zu dem Sondenelement und eine feste räumliche Beziehung relativ zu dem Gehäuse aufzuweisen, wobei der Kraftsensor mit dem Sondenelement verbunden ist; und
einer Positionserfassungsvorrichtung (150), die mit der Elastographievorrichtung verbunden ist;
wobei der Kraftsensor konfiguriert ist, um ein Signal zu erzeugen, das bezeichnend für eine auf das Sondenelement ausgeübte Kraft bei einer Einführung des Sondenelements in ein Material ist, und das Elastographiesystem ferner eine mit dem Kraftsensor verbundene Verarbeitungseinheit (170) umfasst, wobei die Verarbeitungseinheit konfiguriert ist, um, basierend auf Änderungen in dem Signal, eine Abbildung räumlicher Variationen einer Materialeigenschaft innerhalb des Materials als Reaktion auf die Variationen der Materialeigenschaft während des Durchquerens des Sondenelements entlang eines Einführungspfads durch das Material abzuleiten.

**2.** Elastographiesystem nach Anspruch 1, wobei das Sondenelement eine Nadel, ein Schlauch oder ein Draht ist.

**3.** Elastographiesystem nach einem der Ansprüche 1 oder 2, wobei der Kraftsensor ein piezoelektrischer Kristall, ein Dehnungsmessstreifen oder ein Wegsensor ist.

**4.** Elastographiesystem nach einem der Ansprüche 1, 2 oder 3, wobei die Positionserfassungsvorrichtung ein kodierter Objekttisch ist oder wobei die Positionserfassungsvorrichtung ein Ultraschallwegsensor, ein magnetischer Wegsensor oder ein elektrischer Wegsensor ist.

**5.** Elastographiesystem nach einem der Ansprüche 1 bis 4, wobei das Material ein biologisches Gewebe ist und die Verarbeitungseinheit konfiguriert ist, um, basierend auf der Abbildung einen Hinweis auf die Wahrscheinlichkeit einer Unregelmäßigkeit des biologischen Gewebes abzuleiten oder wobei das Materials ein biologisches Gewebe ist, und die Verarbeitungseinheit konfiguriert ist, um, basierend auf der Abbildung einen Hinweis auf die Wahrscheinlichkeit von Krebs abzuleiten, oder wobei das Material ein biologisches Gewebe ist, und die Verarbeitungseinheit konfiguriert ist, um, basierend auf der Abbildung einen Hinweis auf einen Grad oder ein Stadium von Krebs abzuleiten.

**6.** Elastographiesystem nach einem der Ansprüche 1 bis 5, wobei das Sondenelement eines von einer Feinnadelaspirations(FNA)-Nadel oder einer Feinnadelbiopsie(FNB)-Nadel ist.

**7.** Tragbares Elastrographiesystem (100), umfassend:

ein Gehäuse (140);
eine Nadel (120), die entfernbar an dem Gehäuse (140) angebracht ist;
einen Kraftsensor (130), der innerhalb des Gehäuses angeordnet und relativ zu der Nadel fest gelagert ist, wobei der Kraftsensor konfiguriert ist, um eine auf die Nadel ausgeübte Kraft zu erkennen; und
eine Verarbeitungseinheit (170), die mit dem Kraftsensor verbunden ist, wobei die Verarbeitungseinheit konfiguriert ist, um eine Abbildung von räumlichen Variationen einer Materialeigenschaft innerhalb eines biologischen Gewebes basierend auf Änderungen in der Kraft, die an dem Kraftsensor erkannt wird, als Reaktion auf die Variationen der Materialeigenschaft während eines Durchquerens der Nadel entlang eines Einführungspfads durch das biologische Gewebe, abzuleiten, und die Verarbeitungseinheit konfiguriert ist, um, basierend auf der Abbildung einen Hinweis auf die Wahrscheinlichkeit einer Unregelmäßigkeit des biologischen Gewebes abzuleiten.

**8.** Elastographiesystem nach Anspruch 7, ferner umfassend eine Positionserfassungsvorrichtung, die an dem Gehäuse angebracht ist, wobei die Verarbeitungseinheit mit der Positionserfassungsvorrichtung gekoppelt und konfiguriert ist, um eine Trajektorie der Nadel basierend auf Informationen von der Positionserfassungsvorrichtung anzupassen.

**9.** Elastographiesystem nach einem der Ansprüche 7 oder 8, wobei der Kraftsensor eine piezoelektrische Vorrichtung ist, ferner umfassend:

einen Ladungsverstärker, der mit dem Kraftsensor gekoppelt ist, um angesammelte Ladung zu erkennen, wobei der Ladungsverstärker ferner mit der Verarbeitungseinheit gekoppelt ist, und eine Spannung ausgibt, die der Parameter ist, der die an dem Kraftsensor erkannte Kraft darstellt.

**10.** Elastographiesystem nach einem der Ansprüche 7, 8 oder 9, ferner umfassend:
eine serielle Schnittstelle, die mit der Verarbeitungseinheit gekoppelt und konfiguriert ist, um mit Vorrichtungen außerhalb des Gehäuses zu kommunizieren.

**11.** Elastographiesystem nach einem der Ansprüche 7 bis 10, wobei die Nadel eine Feinnadelaspirations(FNA)-Nadel ist.

**12.** Elastographiesystem nach einem der Ansprüche 7 bis 11, wobei der Kraftsensor konfiguriert ist, um eine auf die Nadel ausgeübte axiale und seitliche Kraft zu erkennen.

**Revendications**

**1.** Système d'élastographie (100) comprenant :
un dispositif d'élastographie portatif (110) comportant :

un boîtier (140) ;
un élément de sonde (120) fixé de manière amovible au boîtier ; et
un capteur de force (130) fixé à l'intérieur du boîtier de manière à avoir une relation spatiale fixe par rapport à l'élément de sonde et une relation spatiale fixe par rapport au boîtier, le capteur de force étant relié à l'élément de sonde ; et
un dispositif de détection de position (150) connecté au dispositif d'élastographie ;

le capteur de force étant configuré pour produire un signal indiquant une force appliquée à l'élément de sonde lors de l'insertion de l'élément de sonde dans un matériau, et le système d'élastographie comprenant en outre une unité de traitement (170) connectée au capteur de force, l'unité de traitement étant configurée pour, sur la base des changements dans le signal, dériver une cartographie des variations spatiales d'une propriété de matériau dans le matériau en réponse aux variations de la propriété de matériau pendant la traversée de l'élément de sonde le long d'un chemin d'insertion à travers le matériau.

**2.** Système d'élastographie selon la revendication 1, l'élément de sonde étant une aiguille, un tube ou un fil.

**3.** Système d'élastographie selon l'une quelconque des revendications 1 ou 2, le capteur de force étant un cristal piézoélectrique, une jauge de contrainte ou un capteur de déplacement.

**4.** Système d'élastographie selon l'une quelconque des revendications 1, 2 ou 3, le dispositif de détection de position étant un étage codé, ou le dispositif de détection de position étant un capteur de déplacement à ultrasons, un capteur de déplacement magnétique ou un capteur de déplacement électrique.

**5.** Système d'élastographie selon l'une quelconque des revendications 1 à 4, le matériau étant un tissu biologique, et l'unité de traitement étant configurée pour, sur la base de la cartographie, dériver une indication de probabilité d'une irrégularité du tissu biologique, ou le matériau étant un tissu biologique, et l'unité de traitement étant configurée pour, sur la base de la cartographie, dériver une indication de probabilité de cancer, ou le matériau étant un tissu biologique, et l'unité de traitement étant configurée pour, sur la base de la cartographie, dériver une indication d'un grade ou d'un stade de cancer.

**6.** Système d'élastographie selon l'une quelconque des revendications 1 à 5, l'élément de sonde étant soit une aiguille d'aspiration à l'aiguille fine (FNA), soit une aiguille de biopsie à l'aiguille fine (FNB).

**7.** Système d'élastographie portatif (100) comprenant :

un boîtier (140) ;
une aiguille (120) fixée de manière amovible au boîtier (140) ;
un capteur de force (130) positionné à l'intérieur du boîtier et monté de manière fixe par rapport à l'aiguille, le capteur de force étant configuré pour détecter une force appliquée à l'aiguille ; et
une unité de traitement (170) connectée au capteur de force, l'unité de traitement étant configurée pour dériver une cartographie des variations spatiales d'une propriété de matériau dans un tissu biologique sur la base des changements de la force détectée au niveau du capteur de force, en réponse aux variations de la propriété de matériau pendant une traversée de l'aiguille le long d'un chemin d'insertion à travers le tissu biologique, et l'unité de traitement étant configurée pour, sur la base de la cartographie, dériver une indication de probabilité d'une irrégularité du tissu biologique.

8. Système d'élastographie selon la revendication 7, comprenant en outre un dispositif de détection de position fixé au boîtier, l'unité de traitement étant couplée au dispositif de détection de position et étant configurée pour régler une trajectoire de l'aiguille sur la base des informations provenant du dispositif de détection de position.

9. Système d'élastographie selon l'une quelconque des revendications 7 ou 8, le capteur de force étant un dispositif piézoélectrique, comprenant en outre :
un amplificateur de charge couplé au capteur de force pour détecter la charge accumulée, l'amplificateur de charge étant en outre couplé à l'unité de traitement et fournissant une tension qui est le paramètre représentant la force détectée au niveau du capteur de force.

10. Système d'élastographie selon l'une quelconque des revendications 7, 8 ou 9, comprenant en outre :
une interface série couplée à l'unité de traitement et configurée pour communiquer avec des dispositifs extérieurs au boîtier.

11. Système d'élastographie selon l'une quelconque des revendications 7 à 10, l'aiguille étant une aiguille d'aspiration à l'aiguille fine (FNA).

12. Système d'élastographie selon l'une quelconque des revendications 7 à 11, le capteur de force étant configuré pour détecter une force axiale et latérale appliquée à l'aiguille.

100

110

FNE Device

120

Probing Element

150

Position Sensing Device

130

Force Sensor

160

Sensor Interface

140

Housing Unit

170

Processing Unit

FIG. 1

200

210

Processor

Memory

220

250

Bus

240

230

Communications
Interface

Input/Output Interface

Input
Devices

Output
Devices

FIG. 2

FIG. 3A

FIG. 3B

FIG. 3C

FIG. 4

$Y = 4.2976E-9X-2.1932E-11$
$R^2 = 0.98167$

FIG. 5

FIG. 6

FIG. 7A

FIG. 7B

FIG. 7C

FIG. 7D

FIG. 8A

FIG. 8B

FIG. 9A

FIG. 9B

FIG. 10A

FIG. 10B

FIG. 11

FIG. 12A

FIG. 12B

FIG. 12C

FIG. 12D

FIG. 13A

1340    1345    1330    1355

Position Sensors → Microprocessor

Charge Amplifier ──── USB

1335    1350

Piezo    Needle

Tablet
• Display
• Processing
• Cellular
• Battery
• Memory

1320    1325

FIG. 13B

FIG. 14A

FIG. 14B

FIG. 15

FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62035976, Gimzewski
- WO 2009029627 A1

- WO 2014097301 A1